# EUROPÄISCHE PATENTSCHRIFT

(11) **EP 2 076 303 B1**
(45) Veröffentlichungstag und Bekanntmachung des Hinweises auf die Patenterteilung: **18.07.2018**
(21) Anmeldenummer: 07720140.8
(22) Anmeldetag: 11.05.2007
(51) Int. Cl.: A61M 5/315, A61M 5/24, A61M 5/20

(54) **INJEKTIONSGERÄT MIT MEHREREN KUPPLUNGEN**
INJECTION DEVICE COMPRISING SEVERAL COUPLING MECHANISMS
APPAREIL D'INJECTION À PLUSIEURS ACCOUPLEMENTS

(30) Priorität: 15.09.2006 CH 14752006
(43) Veröffentlichungstag der Anmeldung: 08.07.2009
(73) Patentinhaber: TecPharma Licensing AG, 3400 Burgdorf (CH)
(72) Erfinder: KOHLBRENNER, Philippe, 3413 Kaltacker (CH); STETTLER, Peter, 3422 Kirchberg (CH); HOSTETTLER, Patrick, 3415 Hasle-Rüegsau (CH); WITTMANN, Juergen, 3400 Burgdorf (CH); WITTWER, Martin, 3533 Bowil (CH)
(86) Internationale Anmeldenummer: PCT/CH2007/000243
(87) Internationale Veröffentlichungsnummer: WO 2008/031239

(56) Entgegenhaltungen:
- EP-A- 1 516 638
- EP-A- 1 681 070
- EP-A1- 0 554 995
- WO-A-00/41754
- WO-A-01/19434
- WO-A-2006/039930
- CH-A5- 617 857
- DE-A1-102004 063 644
- US-A1- 2005 197 626
- US-A1- 2005 261 634

## Beschreibung

### Technisches Gebiet

Die vorliegende Erfindung betrifft eine Vorrichtung zur Verabreichung eines fluiden Produkts. Die Vorrichtung kann als Injektionsgerät zur Injektion einer einstellbaren Dosis des Produkts ausgestaltet sein und insbesondere die Form eines Injektionspens, d.h. eines kompakten Injektionsgeräts in Stiftform, annehmen.

### Stand der Technik

Aus dem Stand der Technik sind eine grosse Zahl von Injektionsgeräten zur dosierten Verabreichung von Medikamenten wie Insulin, Wachstumshormonen oder Osteoporosepräparaten bekannt, welche regelmässig verabreicht werden müssen. Derartige Geräte sollen einerseits zuverlässig und präzise eine voreinstellbare Dosis ausschütten. Andererseits sollen sie in hohem Masse bedienungssicher und benutzerfreundlich sein. Dies gilt umso mehr, da sie in der Regel vom Patienten selbst bedient werden.

Das Medikament kann in einer auswechselbaren Karpule untergebracht sein, die in einen Karpulenhalter einsetzbar ist. Dieser lässt sich dann an einem Gehäuse des Injektionsgeräts befestigen, z.B. durch eine Schraubverbindung oder eine Bajonettverbindung. Zur Ausschüttung wird ein Produktstopfen in der Karpule durch eine Fördereinrichtung mit einem Förderelement in Form einer Kolbenstange vorgeschoben.

Insbesondere sind kompakte, stiftförmige Verabreichungsgeräte bekannt, bei denen die Ausschüttung nach einem ersten Auslösen automatisch erfolgt ("Power-assisted pens"). Die Dosis wird bei solchen Geräten in der Regel durch eine Drehung an einem Dosierknopf voreingestellt. Im Gerät ist ein Antrieb vorhanden, z.B. ein Federantrieb, der beim Einstellen der Dosis gespannt wird. Das Gerät wird durch das Eindrücken eines Auslöseknopfs, der mit dem Dosierknopf identisch sein kann, ausgelöst. Dabei erzeugt der Antrieb eine Antriebsbewegung, z.B. in Form einer Drehbewegung, die in eine Vortriebsbewegung der Kolbenstange umgewandelt wird. Im Falle eines Antriebs durch eine Drehbewegung ist die Kolbenstange meist als Gewindestange ausgebildet, auf der eine Antriebsmutter läuft.

In der DE-A 10 2004 063 644 sind Injektionspens offenbart. Diese umfassen ein Gehäuse mit einem Reservoir für das Produkt sowie eine Fördereinrichtung zum Fördern des Produkts. Die Fördereinrichtung ist durch eine Antriebseinrichtung antreibbar. In einer Ausführungsform weist die Antriebseinrichtung eine Drehfeder und ein davon drehbar antreibbares Kupplungseingangsglied auf, das mit einem Kupplungsausgangsglied der Fördereinrichtung kuppelbar ist. Ein Auslöseelement, das gleichzeitig auch als Dosierglied dient, ist so mit der Antriebseinrichtung verbunden, dass die Antriebseinrichtung durch eine Drehung des Auslöseelements gespannt wird. Bei dieser Spannbewegung ist die durch das Kupplungseingangsglied und das Kupplungsausgangsglied gebildete Kupplung ausgekuppelt, d.h., die Spannbewegung wird nur auf die Antriebseinrichtung, nicht aber auf die Fördereinrichtung übertragen. Die Antriebseinrichtung wird beim Spannen durch eine Ratschenverbindung mit einem gehäusefesten Element am Zurückdrehen relativ zum Gehäuse gehindert. Durch axiales Eindrücken des Auslöseelements wird zunächst die Verbindung zwischen dem Auslöseelement und der Antriebseinrichtung gelöst, so dass das Auslöseelement leer drehen kann. Anschliessend wird die Kupplung eingekuppelt und so eine Verbindung zwischen der Antriebseinrichtung und der Fördereinrichtung hergestellt. Schliesslich wird die Ratschenverbindung zwischen der Antriebseinrichtung und dem Gehäuse gelöst, so dass die Antriebseinrichtung nun eine Drehung ausführen kann und dadurch die Fördereinrichtung antreiben kann.

An dieser Anordnung ist nachteilig, dass bei nicht vollständigem Eindrücken des Auslöseelements nur die Verbindung zwischen dem Auslöseelement und der Antriebseinrichtung gelöst wird, ohne dass aber eine Verabreichung ausgelöst wird. Dies ist ein undefinierter Betriebszustand. Der Benutzer kann in diesem Zustand das Auslöseelement, das ja gleichzeitig auch als Dosierglied dient, verdrehen, ohne dass dies irgendeinen Einfluss auf die Verabreichung hätte. Ein solcher undefinierter Zustand kann zu Fehlbedienungen führen; einen solchen Zustand gilt es daher zu vermeiden. Des weiteren sind bei dieser Anordnung notwendig drei Phasen nötig, um eine Verabreichung auszulösen: (a) Freigeben des Auslöse- und Dosierelements; (b) Einkuppeln der Kupplung zum Verbinden von Antriebseinrichtung und Fördereinrichtung; und (c) Lösen der Ratschenverbindung zwischen Antriebseinrichtung und Gehäuse. Keine dieser Phasen kann entfallen, ohne die Funktionalität des Geräts zu zerstören. Konkret werden in dem bekannten Injektionsgerät sogar noch weitere Verbindungen hergestellt und gelöst. Entsprechend bedingt dies einen relativ komplizierten Aufbau des Injektionsgeräts.

### Darstellung der Erfindung

Es ist daher eine Aufgabe der vorliegenden Erfindung, ein Injektionsgerät anzugeben, das einen einfacheren Aufbau aufweist und einen vereinfachten Bewegungsablauf bei der Auslösung einer Injektion ermöglicht. Es ist eine weitere Aufgabe, ein Injektionsgerät anzugeben, bei dem undefinierte Betriebszustände der oben genannten Art vermieden werden.

Diese Aufgaben werden durch den Gegenstand des Anspruchs 1 gelöst. Vorteilhafte Weiterbildungen sind in den abhängigen Ansprüchen angegeben.

Es wird also eine Vorrichtung zur Verabreichung eines fluiden Produkts vorgeschlagen, die die folgenden Einheiten aufweist:
- ein Gehäuse;
- eine Fördereinrichtung zum Fördern des Produkts aus einem Reservoir, wobei die Fördereinrichtung ein drehbares Eingangsglied und ein entlang einer Vorschubachse bewegbares Förderelement umfasst, welches aufgrund einer Drehung des Eingangsglieds antreibbar, insbesondere linear vorschiebbar ist,
- eine Antriebseinrichtung zum Erzeugen einer Antriebsdrehbewegung um die Vorschubachse relativ zum Gehäuse, wobei die Antriebseinrichtung durch eine Spanndrehbewegung spannbar ist,
- eine drehbare Dosiereinrichtung zum Einstellen einer zu verabreichenden Dosis des Produkts und zum Spannen der Antriebseinrichtung; und
- eine Auslöseeinrichtung, die zum Auslösen einer Verabreichung von einer Ruhestellung in eine Auslösestellung bewegbar ist.

Die Vorrichtung umfasst zwei Kupplungen. Jede der Kupplungen kann eine eingekuppelte und eine ausgekuppelte Stellung einnehmen.
- Eine erste Kupplung ist zwischen der Antriebseinrichtung und dem Eingangsglied der Fördereinrichtung ausgebildet. In einer eingekuppelten Stellung dieser Kupplung sind die Antriebseinrichtung und das Eingangsglied der Fördereinrichtung drehfest miteinander verbunden, während diese Teile in einer ausgekuppelten Stellung voneinander gelöst sind.
- Eine zweite Kupplung ist zwischen der Dosiereinrichtung und der Antriebseinrichtung ausgebildet. In einer eingekuppelten Stellung dieser Kupplung sind die Dosiereinrichtung und die Antriebseinrichtung drehfest miteinander verbunden und in einer ausgekuppelten Stellung voneinander gelöst.

Die Kupplungen sind durch die Auslöseeinrichtung bedienbar. In der Ruhestellung der Auslöseeinrichtung nimmt die erste Kupplung ihre ausgekuppelte Stellung und die zweite Kupplung ihre eingekuppelte Stellung ein. Auf diese Weise ist eine Drehung der Dosiereinrichtung auf die Antriebseinrichtung, aber nicht auf die Fördereinrichtung übertragbar. Dadurch wird ein Spannen der Antriebseinrichtung durch eine Drehung der Dosiereinrichtung ermöglicht, ohne dass dabei die Fördereinrichtung bewegt wird. Die Dosiereinrichtung ist dabei vorzugsweise lösbar drehmomentenfest gegenüber dem Gehäuse fixierbar, bevorzugt über eine Ratschenverbindung, d.h. eine durch Drehung der Dosiereinrichtung in wenigstens eine Richtung (der Dosierrichtung) federnd lösbare Rastverbindung, die in mehreren vordefinierten Winkelstellungen einrastet.

Eine Bewegung der Auslöseeinrichtung von der Ruhestellung in die Auslösestellung bewirkt sodann zuerst ein Einkuppeln der ersten Kupplung und danach oder gleichzeitig ein Auskuppeln der zweiten Kupplung. Das Einkuppeln der ersten Kupplung bewirkt, dass die Antriebseinrichtung mit der Fördereinrichtung gekoppelt wird. Es ist aber noch keine Antriebsbewegung möglich, da die Antriebseinrichtung noch über die zweite Kupplung von der Dosiereinrichtung gehalten ist. Erst nachdem die zweite Kupplung ausgekuppelt ist, ist die Antriebseinrichtung freigegeben, und die resultierende Antriebsbewegung ist auf die Fördereinrichtung übertragbar.

Die vorgeschlagene Anordnung ermöglicht also ein definiertes Zusammenspiel der Dosiereinrichtung, der Antriebseinrichtung und der Fördereinrichtung. Indem zuerst die erste Kupplung eingekuppelt und erst dann die zweite Kupplung gelöst wird, wird vermieden, dass undefinierte Betriebszustände auftreten können, bei denen eine der Einrichtungen drehbar ist, ohne dass dies auf den Betrieb der Vorrichtung irgendeinen Einfluss hätte. Des weiteren werden die Bewegungsabläufe vereinfacht, weil im Kern nur zwei Bewegungsphasen notwendig sind, um eine Verabreichung auszulösen.

Wenn die Dosiereinrichtung über eine Ratschenverbindung relativ zum Gehäuse fixierbar ist, ist diese bevorzugt als Doppelrutschkupplung ausgebildet, die ein manuelles Verdrehen der Dosiereinrichtung relativ zum Gehäuse sowohl in einem ersten Drehsinn zum Erhöhen der zu verabreichenden Dosis als auch in einem entgegengesetzten Drehsinn zum Verringern der Dosis ermöglicht. Auf diese Weise ist eine sehr einfache Dosiskorrektur möglich. Dazu weist die Ratschenverbindung bevorzugt eine federbelastete Verzahnung zwischen einem zur Dosiereinrichtung drehfesten Element und einem zum Gehäuse drehfesten Element auf. Da diese Verzahnung im gespannten Zustand des Injektionsgeräts das Drehmoment der Antriebseinrichtung aufnehmen muss, ist die Verzahnung vorzugsweise asymmetrisch ausgestaltet, so dass für ein Lösen der Ratschenverbindung beim Zurückdrehen der Dosiereinrichtung gegen die Dosierrichtung ein grösseres Drehmoment erforderlich ist als für ein Lösen der Verbindung in der Dosierrichtung. Insgesamt kann so erreicht werden, dass der Benutzer des Injektionsgeräts beim Aufdosieren und bei einer Dosiskorrektur ein vergleichbares Drehmoment aufbringen muss.

In einer konkreten Ausgestaltung wird die Ratschenverbindung durch eine axiale Verzahnung zweier Ratschenelemente gebildet, d.h. die Ratschenverbindung umfasst zwei sich axial gegenüberstehende Ratschenelemente, an deren Stirnseiten Zähne ausgebildet sind. Dabei kann ein erstes der Ratschenelemente als axial beweglicher, Richtung des zweiten Ratschenelements federbelasteter Ratschenring ausgestaltet sein, der drehfest zum Dosierelement angeordnet ist, und das zweite Ratschenelement kann dreh- und verschiebefest mit dem Gehäuse verbunden sein. Um Platz zu sparen, kann der Ratschenring durch eine Mehrzahl von entlang dem Umfang des Ratschenrings angeordneten Federn federbelastet sein. Dadurch resultiert ein geringerer Platzbedarf als bei einer Ausführung, bei der der Ratschenring beispielsweise durch eine sich um die Vorschubasche herum erstreckende Schraubenfeder federbelastet ist.

Um die Betriebssicherheit zu erhöhen, kann eine optionale dritte Kupplung vorgesehen werden. Diese ist vorzugsweise zwischen dem Gehäuse und der Fördereinrichtung ausgebildet. In einer eingekuppelten Stellung ist die Fördereinrichtung drehfest mit dem Gehäuse (unmittelbar oder über ein zum Gehäuse drehfestes Element) verbunden. Dadurch verhindert die dritte Kupplung in der eingekuppelten Stellung jede Drehbewegung der Fördereinrichtung und eine dadurch verursachte, unbeabsichtigte Verabreichung des Produkts. In einer ausgekuppelten Stellung sind die Fördereinrichtung und das Gehäuse dagegen voneinander gelöst, so dass in dieser Stellung eine Bewegung der Fördereinrichtung und damit eine Verabreichung des Produkts möglich wird. In der Ruhestellung der Auslöseeinrichtung nimmt die dritte Kupplung ihre eingekuppelte Stellung ein und verhindert so eine unbeabsichtigte Bewegung der Fördereinrichtung. Eine Bewegung der Auslöseeinrichtung von der Ruhestellung in die Auslösestellung bewirkt zuerst ein Einkuppeln der ersten Kupplung, danach ein Auskuppeln der zweiten Kupplung und schliesslich ein Auskuppeln der dritten Kupplung. Dadurch stellt die dritte Kupplung eine wirksame Sicherung gegen eine unbeabsichtigte Verabreichung dar, die erst dann freigegeben wird, wenn das Gerät ansonsten vollständig bereit ist, eine Verabreichung zu bewirken.

Die Verabreichung erfolgt bevorzugt, indem die Auslöseeinrichtung axial gegenüber dem Gehäuse in eine distale Richtung gedrückt wird. Die Auslöseeinrichtung ist dazu bevorzugt entlang der Vorschubachse zwischen der Ruhestellung und der Auslösestellung bewegbar. Jede der Kupplungen umfasst ein Kupplungseingangsglied und ein Kupplungsausgangsglied. Die Auslöseeinrichtung bewirkt durch ihre axiale Verschiebung entlang der Vorschubachse eine relative Verschiebung dieser Glieder, wodurch diese gegeneinander in Eingriff bzw. ausser Eingriff bringbar sind. Bevorzugt ist die Auslöseeinrichtung als Druckknopf ausgebildet, der entlang der Vorschubachse in das Gehäuse hinein eindrückbar ist. Es ist aber auch denkbar, dass die Auslöseeinrichtung z.B. eine Hülse umfasst, die das proximale Ende des Gehäuses zumindest teilweise entlang der Umfangsrichtung umgibt.

Jede der Kupplungen wird vorzugsweise durch Längsnuten und Längsrippen auf radialen Innen- bzw. Aussenflächen des jeweiligen Kupplungseingangsglieds bzw. Kupplungsausgangsglieds gebildet. Diese Längsnuten und Längsrippen lassen sich durch gegenseitige Längsverschiebung der Kupplungseingangs- und Kupplungsausgangsglieder in und ausser Eingriff nach Art einer Nut-Feder-Verbindung bringen. Eine solche Anordnung ist gegenüber anderen denkbaren Formen, z.B. stirnseitigen Verzahnungen oder Schrägverzahnungen, sehr platzsparend, und sie ermöglicht es, dass sich beispielsweise ein Kupplungseingangsglied sowohl in proximaler als auch in distaler Richtung über das entsprechende Kupplungsausgangsglied hinaus erstreckt.

In einer bevorzugten Ausführungsform ist die gesamte Antriebseinrichtung entlang der Vorschubachse verschiebbar angeordnet, so dass die Bewegung der Auslöseeinrichtung von der Ruhestellung in die Auslösestellung eine distale Verschiebung der Antriebseinrichtung aus ihrer Ausgangsstellung heraus bewirkt. Dies ermöglicht eine kompakte Ausgestaltung des Injektionsgeräts. Dabei ist vorzugsweise die Antriebseinrichtung entlang der Vorschubachse in proximaler Richtung federbelastet, um eine automatische Rückkehr der Antriebseinrichtung in ihre Ausgangsstellung am Ende der Verabreichung sicherzustellen. Weitere Federn zu diesem Zweck können entfallen, da die federbelastete Antriebseinrichtung gleichzeitig auch die Auslöseeinrichtung in die Ruhestellung zurückdrückt.

Bevorzugt umfasst die Vorrichtung einen lösbaren Behältnishalter zum Halten eines Reservoirs mit dem Produkt. Das Förderelement ist entlang der Vorschubachse zwischen einer proximalen Ausgangsstellung und einer distalen Endstellung bewegbar. Ein Dosisbegrenzungselement ist zwischen einer Ausgangsstellung und einer Dosisbegrenzungsstellung bewegbar und wirkt mit der Dosiereinrichtung derart zusammen, dass es ein Einstellen einer Dosis verhindert, deren Verabreichung eine Bewegung des Förderelements über seine distale Endstellung hinaus erfordern würde, indem es in der Dosisbegrenzungsstellung einen Anschlag bildet. Da die distale Endstellung des Förderelements in der Regel einer Situation entspricht, in der das Reservoir bis auf eine unvermeidbare Restmenge vollständig entleert ist, verhindert das Dosisbegrenzungselement also, dass an der Dosiereinrichtung eine Dosis eingestellt wird, die die verfügbare Menge des Produkts überschreitet. Bei einem Reservoirwechsel verschiebt sich das Förderelement selbsttätig in die distale Endstellung. Dazu umfasst die Vorrichtung ein Federelement, welches eine Federkraft erzeugt, die auf das Förderelement entlang der Vorschubachse in die distale Richtung gegenüber dem Gehäuse wirkt und eine Bewegung des Förderelements in seine distale Endstellung bewirkt, wenn der Behältnishalter vom Gehäuse gelöst wird. Gleichzeitig wird das Dosisbegrenzungselement selbsttätig in seine Dosisbegrenzungsstellung gebracht. Dazu ist das Dosisbegrenzungselement zumindest in einem Zustand, in dem der Behältnishalter vom Gehäuse gelöst ist, derart mit dem Förderelement gekoppelt, dass die Bewegung des Förderelements in seine distale Endstellung eine Bewegung des Dosisbegrenzungselements in seine Dosisbegrenzungsstellung bewirkt. Nachdem der Behältnishalter vom Gehäuse gelöst wurde, befinden sich also das Förderelement in seiner distalen Endstellung und das Dosisbegrenzungselement in seiner Dosisbegrenzungsstellung. Wenn in diesem Zustand das Förderelement in Richtung seiner proximalen Endstellung bewegt wird, bleibt das Dosisbegrenzungselement weiterhin mit dem Förderelement gekoppelt, so dass diese Bewegung des Förderelements eine proportionale Bewegung des Dosisbegrenzungselements in Richtung seiner Ausgangsstellung bewirkt.

Beim Einsetzen eines neuen Reservoirs wird der Stopfen des Reservoirs das Förderelement in das Gehäuse einschieben, und zwar um einen Betrag, der gerade der verfügbaren Produktmenge im Reservoir entspricht. Durch die Kopplung mit dem Dosisbegrenzungselement wird auch dieses um einen entsprechenden Betrag bewegt, so dass nach dem Einsetzen des Reservoirs das Dosisbegrenzungselement um einen Betrag von seiner Dosisbegrenzungsstellung entfernt liegen wird, der genau der verfügbaren Produktmenge entspricht. Auf diese Weise ist einerseits sichergestellt, dass jederzeit eine korrekte Verabreichung erfolgt, auch wenn das Reservoir beim Einsetzen nur teilweise gefüllt war, und dass die Dosisbegrenzungseinrichtung auch in diesem Fall korrekt funktioniert.

Um dies zu ermöglichen, sind vorzugsweise die Kupplungen in einem Zustand, in dem der Behältnishalter vom Gehäuse gelöst ist, ausgekuppelt.

Das Förderelement steht bevorzugt derart mit dem Eingangsglied der Fördereinrichtung in einem Gewindeeingriff, dass eine Bewegung des Förderelements in seine distale Endstellung zu einer Drehbewegung des Eingangsglieds führt. Das Dosisbegrenzungselement ist dann vorzugsweise derart mit dem Eingangsglied gekoppelt, dass die Drehbewegung des Eingangsglieds eine Bewegung des Dosisbegrenzungselements in seine Dosisbegrenzungsstellung bewirkt.

Das Förderelement kann insbesondere ein Innengewinde aufweisen, dessen Gewindeachse entlang der Vorschubachse verläuft. Das Eingangsglied umfasst dann ein Aussengewinde, das mit dem Innengewinde in Eingriff steht. Dadurch kann die Aussenseite des Eingangsglieds so ausgestaltet werden, dass sie einfach gegenüber dem Gehäuse gedichtet werden kann, insbesondere so, dass sie einen im Wesentlichen glatten Bereich aufweist. Zudem kann so das Erscheinungsbild des Injektionsgeräts für einen Benutzer verbessert werden.

Das Dosisbegrenzungselement kann hülsen- oder ringförmig ausgebildet sein. Es ist bevorzugt drehfest und entlang der Vorschubachse verschiebbar relativ zum Eingangsglied angeordnet und steht über ein Gewinde derart mit einem weiteren Element in Eingriff, dass eine Drehung des Dosisbegrenzungselements gleichzeitig zu einer Verschiebung des Dosisbegrenzungselements entlang der Vorschubachse führt, solange das weitere Element stationär zum Gehäuse ist.

Dieses weitere Element ist bevorzugt ein Teil der Antriebseinrichtung, das während der Verabreichung die Antriebsdrehbewegung ausführt. Indem die erste Kupplung während der Verabreichung eingekuppelt ist, erfolgt während der Verabreichung keine Bewegung des Dosisbegrenzungselements entlang der Vorschubachse, da sowohl das Eingangsglied der Fördereinrichtung als auch die Antriebseinrichtung mit gleicher Winkelgeschwindigkeit drehen, während aber ein dem Zustand, in dem der Behältnishalter vom Gehäuse gelöst ist und in dem daher die erste Kupplung gelöst ist, ein Vorschub des Förderelements auch zu einer Verschiebung des Dosisbegrenzungselements führt.

In einer bevorzugten Ausgestaltung umfasst die Vorrichtung ein Mitnahmeelement, das relativ zum Gehäuse drehbar angeordnet ist. Das Mitnahmeelement ist derart gegenüber dem Gehäuse geführt, dass es beim Befestigen des Behältnishalters am Gehäuse sowie beim Lösen des Behältnishalters vom Gehäuse durch den Behältnishalter mitgenommen und in eine Bewegung versetzt wird, die eine Drehbewegung um die Drehachse umfasst. Ein bevorzugt axial durch eine Federelement federbelastetes Rastelement bewirkt dann in der Haltestellung des Behältnishalters eine lösbare Rastverbindung, durch die das Mitnahmeelement relativ zum Gehäuse fixiert wird. Dieses wiederum hält dann den Behältnishalter. Der Behältnishalter wird also indirekt, über das Mitnahmeelement, relativ zum Gehäuse fixiert. Dadurch wird eine grosse Freiheit bei der Gestaltung des Behältnishalters und des Rastelements und Federelements ermöglicht.

In einer vorteilhaften Ausgestaltung umfasst die Vorrichtung ein relativ zum Gehäuse drehfest angeordnetes Führungselement, das insbesondere als Führungshülse ausgebildet sein kann. Dieses Führungselement kann starr mit dem Gehäuse verbunden sein, insbesondere einstückig mit diesem gefertigt sein, oder es kann axial gegenüber dem Gehäuse verschiebbar sein. Das Mitnahmeelement, das ebenfalls als Hülse ausgestaltet sein kann und dann als Bajonetthülse bezeichnet werden kann, ist dann mit dem Führungselement drehbar verbunden. Das Federelement und das Rastelement sind vorzugsweise relativ zum Führungselement drehfest angeordnet, und das Rastelement ist in der Haltestellung des Behältnishalters mit dem Mitnahmeelement lösbar verrastet. Insbesondere kann das Führungselement gegenüber dem Gehäuse axial verschiebbar und das Mitnahmeelement zwar drehbar, aber axial verschiebefest mit dem Führungselement verbunden sein. Durch diese Gestaltung wird ermöglicht, dass weitere im Gehäuse angeordnete Teile der Vorrichtung, die mit dem Führungselement verbunden sind, axial verschoben werden, wenn der Behältnishalter vom Gehäuse gelöst wird. Dadurch kann insbesondere erreicht werden, dass die oben beschriebenen Kupplungen ausgekuppelt werden.

Bevorzugt ist dabei das Mitnahmeelement derart gegenüber dem Gehäuse geführt, dass es beim Befestigen des Behältnishalters am Gehäuse vom Behältnishalter mitgenommen und in eine kombinierte Drehbewegung um die Drehachse und Translationsbewegung entlang der Drehachse in einer proximalen Richtung versetzt wird.

Vorzugsweise ist das Mitnahmeelement zwischen zwei definierten Endstellungen bewegbar, und das Rastelement bewirkt in *beiden* Stellungen eine lösbare Rastverbindung, durch die das Mitnahmeelement relativ zum Gehäuse fixiert wird. Dabei nimmt das Mitnahmeelement seine erste Endstellung ein, wenn der Behältnishalter seine Haltestellung einnimmt, und es nimmt seine zweite Endstellung ein, wenn der Behältnishalter vom Gehäuse abgenommen ist. Bevorzugt ist das Rastelement sowohl in der ersten Endstellung als auch in der zweiten Endstellung unmittelbar mit dem Mitnahmeelement lösbar verrastet.

Das Mitnahmeelement ist vorzugsweise in mindestens einem Führungsschlitz relativ zum Gehäuse, d.h. am eigentlichen Gehäuse selbst oder an einem gehäusefesten Element, geführt, insbesondere durch ein oder mehrere entsprechende Zapfen. Ebenso ist der Behältnishalter beim Befestigen am Gehäuse in mindestens einem Führungsschlitz, nach Art einer Bajonettverbindung, relativ zum Gehäuse geführt.

Das Rastelement ist vorzugsweise in Form eines Rings ausgebildet, der sich um die Drehachse und insbesondere um das Vorschubelement herum erstreckt. Auf dem Ring können geeignete Rastnasen ausgebildet sein. Dieses Rastelement kann durch ein getrenntes Federelement axial federbelastet sein. Dabei kann es sich z.B. um eine auf Druck belastete Schraubenfeder oder eine andere Art von elastischem Element handeln. Bevorzugt weist das Federelement jedoch die Form eines Rings auf, welcher sich um die Drehachse herum erstreckt und um eine Achse senkrecht zur Drehachse gekrümmt ist, so dass durch ein Zusammenpressen des Federelements entlang der Drehachse die Federkraft erzeugt wird.

Bevorzugt ist das Rastelement einstückig mit dem Federelement ausgebildet. Dies führt zu einer besonders einfachen Ausgestaltung. Insbesondere kann das Rastelement als ein in Richtung der Drehachse vorstehender Vorsprung auf dem Federelement ausgebildet sein. Dies ist insbesondere dann von Vorteil, wenn das Federelement, wie oben beschrieben, die Form eines gekrümmten Rings aufweist.

Die Vorrichtung kann des weiteren mindestens ein Kugellager aufweisen, welches Kräfte aufnimmt, die zwischen dem Förderelement und dem Gehäuse übertragen werden. Indem ein Kugellager vorgesehen wird, werden Reibungsverluste, die während der Verabreichung aufgrund der Übertragung von Kräften zwischen dem Förderelement und dem Gehäuse verursacht werden, weitgehend minimiert.

Das Kugellager ist vorteilhaft derart angeordnet, dass es entlang der Vorschubachse wirkende (Axial-) Kräfte aufnimmt, welche vom (vorzugsweise relativ zum Gehäuse drehfesten) Förderelement über das drehbare Element auf das Gehäuse übertragen werden, also zwischen dem drehbaren Element und dem Gehäuse wirken. Allgemein ausgedrückt werden also axiale, insbesondere in proximaler Richtung wirkende Kräfte zwischen dem Förderelement und dem Gehäuse über eine drehbare und in axialer Richtung verschiebefeste Verbindung hinweg übertragen. Im Stand der Technik wird hierzu in der Regel eine gleitende Verbindung zwischen dem drehbaren Element und dem Gehäuse bzw. einem gehäusefesten Element vorgesehen. Bei der vorliegenden Ausgestaltung ist dagegen mindestens ein Kugellager vorgesehen, das bevorzugt zwischen dem drehbaren Element oder einem damit verbundenen Element einerseits und dem Gehäuse oder einem zumindest während der Verabreichung gehäusefesten Element andererseits angeordnet ist. Dadurch können Reibungsverluste bei der Drehung des drehbaren Elements vermieden werden. Bevorzugt sind zwei Kugellager vorhanden, die vorzugsweise so angeordnet sind, dass sie Kräfte entlang der Vorschubachse sowohl in einer proximalen Richtung als auch in der dazu entgegengesetzten distalen Richtung, d.h. der Richtung, in welcher der Vorschub erfolgt, aufnehmen können. Axialkräfte in der proximalen Richtung entstehen in der Regel während der Verabreichung, während Axialkräfte in der distalen Richtung insbesondere dann entstehen können, wenn das Förderelement in der distalen Richtung federbelastet ist, wie dies in einer bevorzugten Ausführungsform der Fall ist. Die dazu dienende Feder ist bevorzugt so schwach, dass sie keinen Ausstoss des Produkts aus dem Reservoir bewirkt. Sie dient vielmehr dazu, bei einem Reservoirwechsel das Förderelement selbsttätig in seine distale Endstellung zu bringen, also vollständig auszufahren, wenn das Reservoir vom Gehäuse entfernt wird. Wenn das Förderelement mit dem drehbaren Element geeigneter Verbindung steht, führt dies dazu, dass das drehbare Element in eine Drehung versetzt wird, wenn das Förderelement aufgrund der Federkraft ausfährt. Um die dazu nötige Federkraft klein halten zu können, ist ein Kugellager von besonderem Vorteil, da es beim Ausfahren des Förderelements die dabei wirkenden Reibungskräfte minimiert.

### Kurze Beschreibung der Zeichnungen

Bevorzugte Ausführungsformen der Erfindung werden im Folgenden anhand der Zeichnungen beschrieben, in denen zeigen:
- Fig. 1: eine perspektivische Explosionsansicht eines Injektionsgeräts gemäss einer ersten Ausführungsform;
- Fig. 2: einen Längsschnitt durch das Injektionsgerät der Fig. 1;
- Fig. 3: einen vergrösserten Ausschnitt aus der Fig. 2;
- Fig. 4: einen Längsschnitt durch eine Anordnung einer Führungshülse und einer Kupplungshülse im Injektionsgerät der Fig. 1;
- Fig. 5A: eine Draufsicht auf einen Kugellagerring;
- Fig. 5B: eine Schnittansicht des Kugellagerrings in der Ebene A-A;
- Fig. 6: eine perspektivische Ansicht einer Kupplungshülse;
- Fig. 7: einen Längsschnitt durch ausgewählte Teile des Injektionsgeräts der Fig.1;
- Fig. 8: eine perspektivische Darstellung einer Bajonetthülse;
- Fig. 9A: eine Draufsicht auf eine Bajonettfeder;
- Fig. 9B: eine Seitenansicht der Bajonettfeder der Fig. 9A;
- Fig. 9C: eine perspektivische Ansicht der Bajonettfeder der Fig. 9A;
- Fig. 10: einen Längsschnitt durch ausgewählte Teile des Injektionsgeräts der Fig. 1;
- Fig. 11A: einen Längsschnitt durch ausgewählte Teile des Injektionsgeräts der Fig. 1 mit einem Dosisbegrenzungsring in seiner Endstellung;
- Fig. 11B: einen Längsschnitt durch ausgewählte Teile des Injektionsgeräts der Fig. 1 mit dem Dosisbegrenzungsring in seiner Ausgangsstellung;
- Fig. 12A: eine perspektivische Ansicht einer Kupplungswelle mit einem Dosisbegrenzungsring des Injektionsgeräts der Fig. 1;
- Fig. 12B: die Teile der Fig. 12A in einer Explosionsansicht;
- Fig. 12C: die Kupplungswelle der Fig. 12A in einer perspektivischen Ansicht aus einer anderen Blickrichtung;
- Fig. 13: eine Explosionsansicht einer Arretierhülse, einer Kupplungsfeder und eines Stützrings;
- Fig. 14: eine perspektivische Ansicht ausgewählter Teile des Injektionsgeräts der Fig. 1;
- Fig. 15: eine perspektivische Explosionsansicht des proximalen Endes des Injektionsgeräts der Fig. 1;
- Fig. 16: einen Längsschnitt durch das Injektionsgerät der Fig. 1 in der Ausgangsstellung;
- Fig. 17: den Längsschnitt der Fig. 16 nach einem ersten Aufdosieren bis zur halben Maximaldosis;
- Fig. 18: den Längsschnitt der Fig. 16 nach einem ersten Aufdosieren bis zur vollen Maximaldosis;
- Fig. 19: den Längsschnitt der Fig. 16 nach einem ersten Auslösen und einem zweiten Aufdosieren;
- Fig. 20: einen Längsschnitt durch das Injektionsgerät der Fig. 1 nach vollständiger Entleerung der Karpule;
- Fig. 21: einen Längsschnitt durch ein Injektionsgerät gemäss einer zweiten Ausführungsform.

### Ausführliche Beschreibung bevorzugter Ausführungsformen

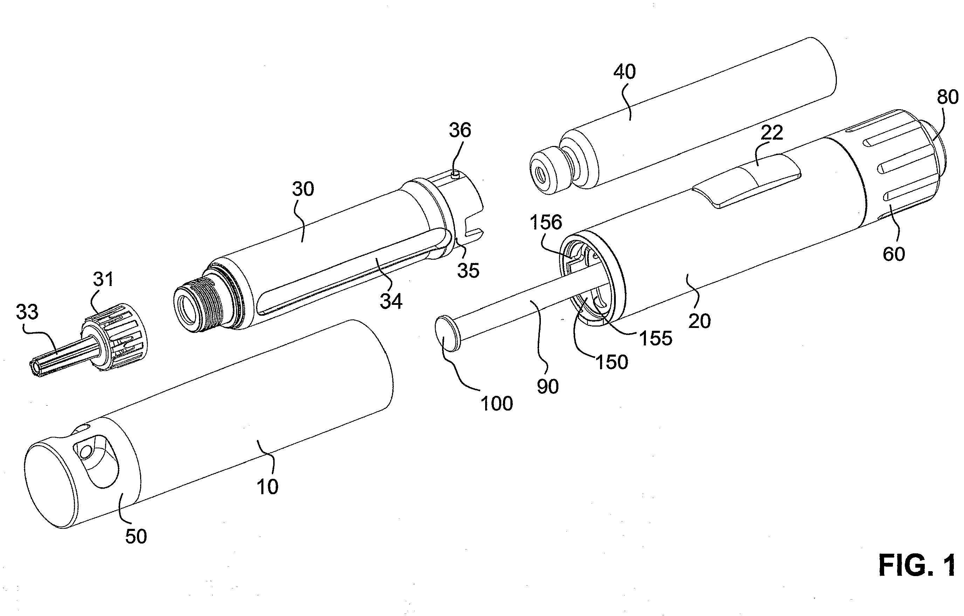

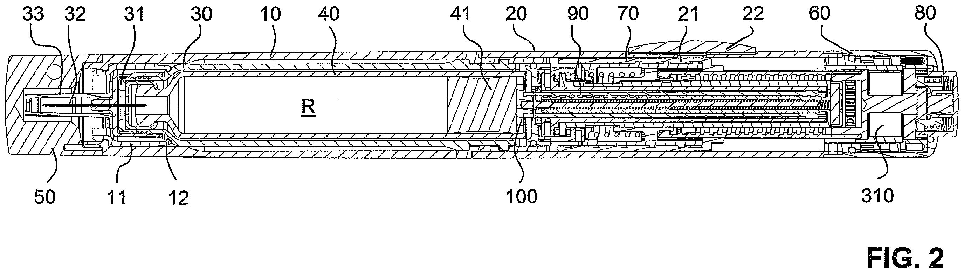

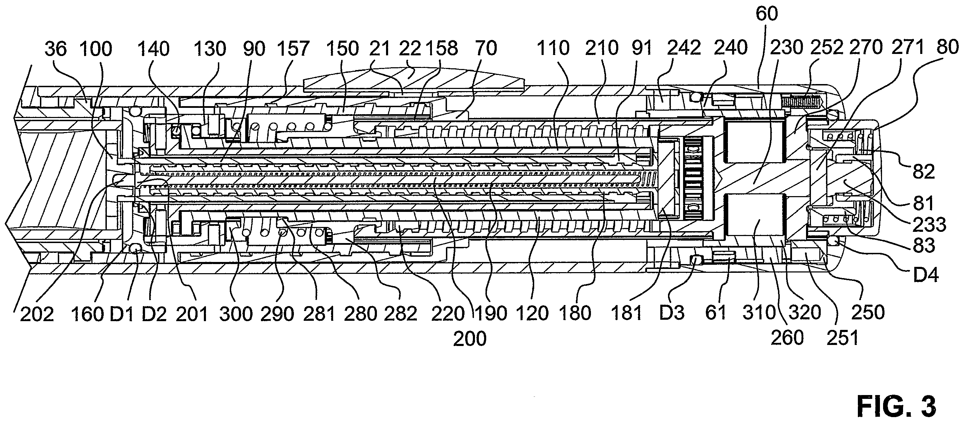

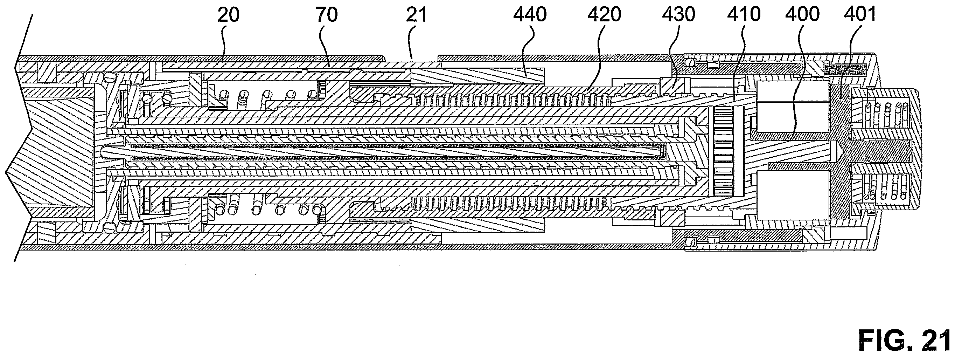

Anhand der Figuren 1 und 2 werden im Folgenden zunächst der grundsätzliche Aufbau und die grundsätzliche Bedienung eines erfindungsgemässen Injektionsgeräts erläutert, bevor für eine erste Ausführungsform der Aufbau des Geräts und die Funktionsweise der Dosiermechanik im Einzelnen anhand der Figuren 3 bis 20 erläutert wird. Eine zweite Ausführungsform wird dann anhand der Fig. 21 diskutiert.

### Grundsätzlicher Aufbau und Bedienung

Die Fig. 1 zeigt ein Injektionsgerät in Form eines Injektionspens in einer perspektivischen Explosionsansicht. Die Fig. 2 zeigt das Gerät im Längsschnitt. Die folgende Beschreibung bezieht sich auf das Gerät im zusammengesetzten Zustand, wie es in der Fig. 2 dargestellt ist.

Das Injektionsgerät weist eine Gehäusehülse 20 auf, in der eine Mechanik zum Einstellen und Ausschütten einer Dosis untergebracht ist. Die Gehäusehülse 20 ist im Wesentlichen kreiszylinderförmig und definiert hierdurch eine Längsachse. Über eine unten näher beschriebene Bajonettverbindung ist ein Behältnishalter in Form einer Karpulenhülse 30 an einem distalen Ende der Gehäusehülse 20 lösbar befestigt. Diese nimmt ein Behältnis in Form einer Karpule 40 mit einem flüssigen Medikament auf, in der ein Stopfen 41 verschiebbar geführt ist. Dadurch wird ein Medikamentenreservoir R veränderlichen Volumens innerhalb der Karpule begrenzt. Statt einer Karpule kann auch ein anderes Behältnis vorhanden sein, dessen Volumen veränderlich ist, z.B. ein Behältnis mit ziehharmonikaartig gefalteten Wänden nach Art eines Faltenbalgs. Durch ein langgestrecktes Sichtfenster 34 in der Karpulenhülse 30 kann der Inhalt der Karpule 40 kontrolliert werden. Am distalen Ende der Karpulenhülse 30 ist ein Nadelhalter 31 aufgeschraubt, der eine als Injektionsnadel dienende Hohlnadel (Kanüle) 32 trägt, deren proximales Ende durch ein dichtendes Septum hindurch in das Medikamentenreservoir R ragt. Eine abziehbare Nadelschutzhülle 33 umgibt den nach vorne ragenden Bereich der Nadel 32 und schützt einen Benutzer vor einem versehentlichen Einstechen. Eine Schutzhülse 10, deren distales Ende permanent mit einer Schutzkappe 50 verschlossen ist, ist über die Karpulenhülse 30 geschoben. Im Inneren der Schutzhülse 10 ist ein Haltering 11 mit sich in proximaler Richtung erstreckenden Rastarmen 12 montiert. Die Enden der Rastarme 12 sind lösbar mit der Karpulenhülse 30 verrastet. Während die Erfindung hier anhand eines Injektionsgeräts erläutert wird, das eine Nadel 32 aufweist, ist es auch denkbar, dass das Injektionsgerät mehrere Nadeln oder keine Nadel wie bei einem Jet-Injektor aufweist.

Am proximalen Ende der Gehäusehülse 20 ist eine Dosierhülse 60 mit darin gehaltenem Druckknopf 80 verdrehbar angeordnet. Die Dosierhülse dient zur Einstellung einer aus dem Medikamentenreservoir R auszuschüttenden Dosis und zum Spannen einer Antriebseinrichtung mit einem Antriebselement in Form einer zeichnerisch nur angedeuteten, als Drehfeder wirkenden Spiralfeder 310. Die eingestellte Dosis wird auf einer Anzeigetrommel 70 angezeigt und kann durch ein Fenster 21 in der Gehäusehülse 20 abgelesen werden, welches durch eine transparente Abdeckung 22 abgedeckt ist. Ein Korrigieren (Verringern) der eingestellten Dosis ist auf einfache Weise durch ein Zurückdrehen der Dosierhülse 60 möglich. Unten wird unter Bezugnahme auf die weiteren Figuren noch im Einzelnen erläutert, wie diese Einstellung ermöglicht wird.

Unter Bezugnahme auf diese Teile lassen sich die folgenden Richtungen definieren, auf die im Folgenden immer wieder Bezug genommen wird: Die distale Richtung ist diejenige Richtung, in der die Verabreichung erfolgt, d.h. sie weist entlang der Längsachse vom Druckknopf 80 in Richtung der Hohlnadel 32. Die proximale Richtung ist entsprechend als die entgegengesetzte Richtung definiert. Falls auf einen Drehsinn (Uhrzeigersinn, Gegenuhrzeigersinn) Bezug genommen wird, so ist damit der Drehsinn gemeint, den man wahrnimmt, wenn man entlang der Längsachse in distaler Richtung blickt.

Nach dem Einstellen der Dosis wird die Hohlnadel 32 durch die Haut des Patienten eingestochen, und es wird eine Ausschüttung der Dosis ausgelöst, indem der Benutzer den Druckknopf 80 in die Dosierhülse 60 eindrückt. Über einen unten im Einzelnen beschriebenen Mechanismus wird durch die Antriebseinrichtung eine Drehbewegung erzeugt, die in einen Vortrieb eines Förderelements in Form einer Vorschubhülse 90 in distaler Richtung umgewandelt wird. Die Vorschubhülse 90 schiebt über einen an ihrem distalen Ende angeordneten Vorschubflansch 100 den Stopfen 41 der Medikamentenkarpule 40 um den eingestellten Betrag in distaler Richtung, wodurch die Ausschüttung des Medikaments aus dem Reservoir R bewirkt wird. Die Vorschubhülse 90 wirkt also als Kolbenstange für den durch den Vorschubflansch 100 und den Stopfen 41 gebildeten Kolben. Nach dem Ende der Verabreichung lässt der Benutzer den Druckknopf 80 wieder los. Die Anzeigetrommel wird während des Vortriebs der Vorschubhülse 90 von der Antriebseinrichtung derart mitgenommen, dass sie im Verlauf der Ausschüttung in ihre Nullstellung zurückkehrt. Damit ist der Injektionspen sofort für die nächste Dosiseinstellung bereit.

Wenn das Medikament im Medikamentenreservoir R zur Neige geht, also die Vorschubhülse 90 beinahe vollständig ausgefahren ist, wird dies durch eine unten näher beschriebene Dosisbegrenzungseinrichtung im Injektionspen erkannt. Die Dosisbegrenzungseinrichtung erlaubt es dem Benutzer dann lediglich noch, maximal die verbleibende verfügbare Restdosis einzustellen. Bei einem anschliessenden Karpulenwechsel stellen sich die Dosisbegrenzungseinrichtung wie auch die Anzeigetrommel 70 automatisch in den Ausgangszustand zurück. Es ist also keine manuelle Rückstellung erforderlich.

Im Folgenden werden Aufbau und Funktionsweise der Mechanik im Einzelnen beschrieben.

### Aufbau der Mechanik

Die Fig. 3 zeigt eine vergrösserte Darstellung des hinteren (proximalen) Bereichs des Injektionsgeräts der Fig. 1. Der Aufbau dieses Bereichs wird nun im Wesentlichen von innen nach aussen im Detail beschrieben.

Die Vorschubhülse 90 ist in einer zum Gehäuse drehfest und verschiebbar angeordneten Führungshülse 110 wiederum drehfest und in Längsrichtung verschiebbar gelagert. Hierzu weist die Vorschubhülse 90 an ihrem proximalen Ende mehrere radial nach aussen ragende Führungsnocken 91 auf, die in dazu komplementären Längsnuten auf der Innenseite der Führungshülse 110 geführt sind.

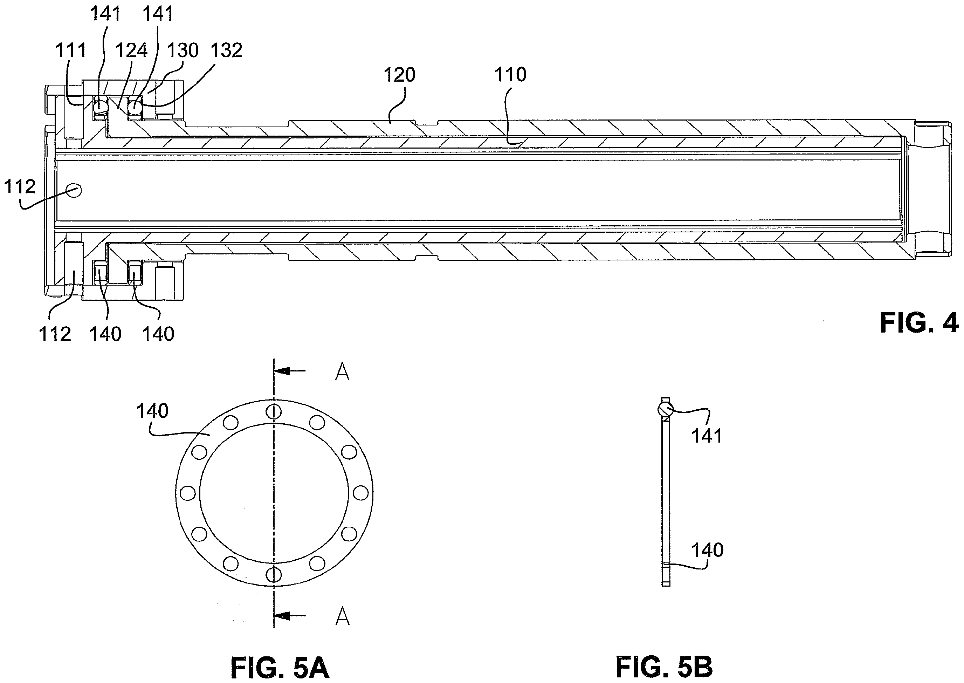

Die Führungshülse 110 ist besonders gut in der Fig. 4 erkennbar, die das Zusammenwirken der Führungshülse mit weiteren Teilen illustriert. Die Führungshülse 110 weist an ihrem distalen Ende einen radial nach aussen ragenden, umlaufenden Ringflansch 111 mit radialen Bohrungen 112 auf. Ein ringförmiger Lagerhalter 130 ist von der proximalen Seite her über die Führungshülse 110 geschoben, umgibt den Ringflansch 111 radial und ist über zeichnerisch nicht dargestellte radiale Zylinderstifte starr mit diesem verbunden.

Eine Kupplungshülse 120 ist zwischen dem Ringflansch 111 der Führungshülse 110 und einer nach innen ragenden Schulter 132 des Lagerhalters 130 drehbar gelagert. Wie unten noch näher beschrieben wird, ist die Kupplungshülse 120 über eine Gewindestange 180 mit der Vorschubhülse 90 verbunden und bildet daher einen Teil einer Fördereinrichtung, die durch eine Drehbewegung angetrieben wird und einen Vorschub des Förderelements in Form der Vorschubhülse bewirkt. Die Kupplungshülse 120 nimmt daher im Betrieb erhebliche Axialkräfte auf, die über ihre Lagerung auf die Führungshülse 110, den Lagerhalter 130, den Mechanikhalter 150 und damit letztlich auf das Gehäuse übertragen werden.

Um die Lagerung möglichst verlustarm zu gestalten, erfolgt die Lagerung über Kugellager. Hierzu ist zwischen dem Flansch 111 der Führungshülse und einem radial umlaufenden Flansch 124 der Kupplungshülse 120 ein erster Kugellagerring 140 vorgesehen. Ein weiterer derartiger Kugellagerring 140 ist zwischen dem Flansch 124 und einer Stirnfläche des Lagerhalters 130 angeordnet.

Der Kugellagerring 140 ist in den Figuren 5A und 5B im Detail dargestellt. Er trägt eine Mehrzahl von Lagerkugeln 141 (hier zwölf Stück). Diese laufen, wie in der Fig. 3 erkennbar ist, in flachen, kreisförmigen Rillen, die in beiden Stirnseiten des radialen Flansches 124 der Kupplungshülse 120, in der entsprechenden Stirnseite des Flansches 111 der Führungshülse 110 sowie in der Stirnseite des Lagerhalters 130 ausgebildet sind.

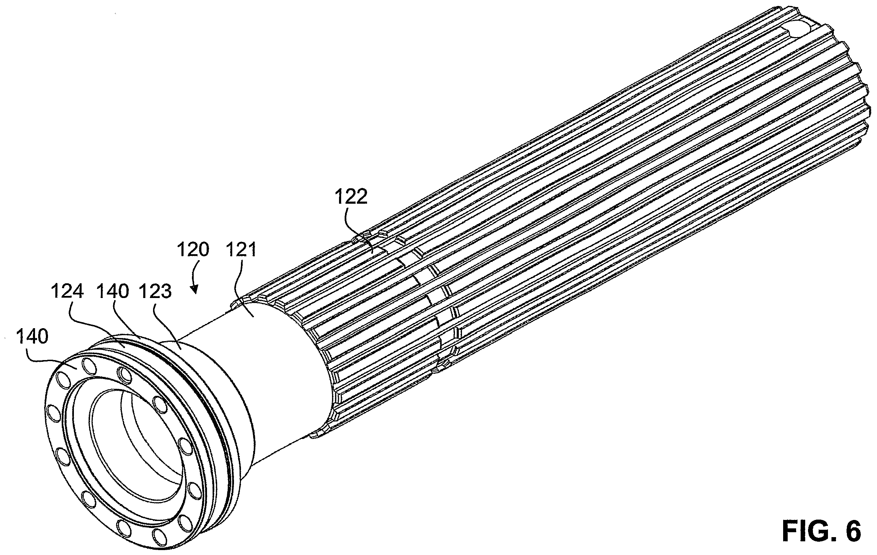

Die Kupplungshülse 120 ist zusammen mit den Kugellagerringen 140 (aber ohne Kugeln 141) in der Fig. 6 dargestellt. Wie hier besonders gut erkennbar ist, sind auf dem zylindrischen Hülsenkörper 121 eine Mehrzahl von Längsrippen 122 ausgebildet, die sich über einen erheblichen Teil der Länge des Hülsenkörpers in Längsrichtung bis zu dessen proximalem Ende erstrecken. Dazwischen sind entsprechende Nuten vorhanden. Auf eine Verdickung 123 folgt nach vorne hin der Flansch 124, der beidseitig an die Kugellagerringe 140 angrenzt.

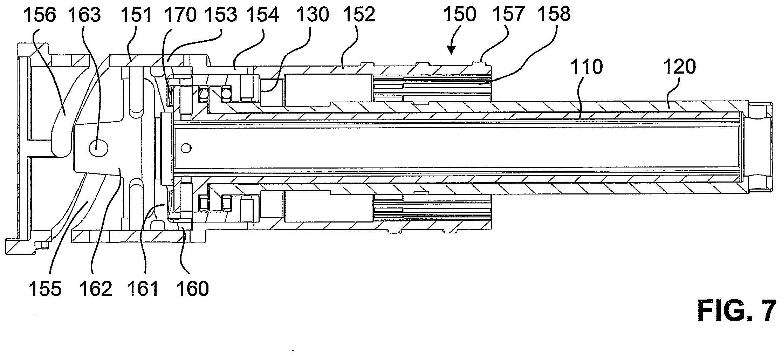

In der Fig. 7 ist dargestellt, wie die Einheit aus Führungshülse 110 und Lagerhalter 130 in einem hülsenförmigen Mechanikhalter 150 drehfest, aber in Längsrichtung verschiebbar gehalten ist.

Der Mechanikhalter 150 weist einen distalen Abschnitt 151 mit vergrössertem Innen- und Aussendurchmesser sowie einen proximalen Abschnitt 152 mit etwas geringerem Innen- und Aussendurchmesser auf. Diese beiden Abschnitte sind durch eine Stufe 153 verbunden. Die Aussenseite des distalen Abschnitts 151 ist starr in der Gehäusehülse 20 gehalten. Damit ist also auch der gesamte Mechanikhalter 150 gegenüber dem Gehäuse unbeweglich, bildet also funktional einen Teil des Gehäuses.

Angrenzend an die Stufe 153 sind im Mechanikhalter 150 mindestens zwei Längsschlitze 154 ausgebildet. Im Lagerhalter 130 sind Zapfen eingesetzt, die in der Fig. 7 nicht dargestellt sind. Diese ragen radial über den Lagerhalter 130 hinaus und in die Längsschlitze 154 des Mechanikhalters hinein. Hierdurch sind der Lagerhalter 130 und die mit diesem fest verbundene Führungshülse 110 in Längsrichtung zwischen einer distalen und einer proximalen Endstellung verschiebbar und drehgesichert im Mechanikhalter 150 geführt. Zum proximalen Ende hin ist auf der äusseren Mantelfläche des Mechanikhalters 150 ein Aussengewinde 157 ausgebildet. Auf der Innenfläche sind in diesem Bereich mehrere Längsnuten 158 ausgebildet.

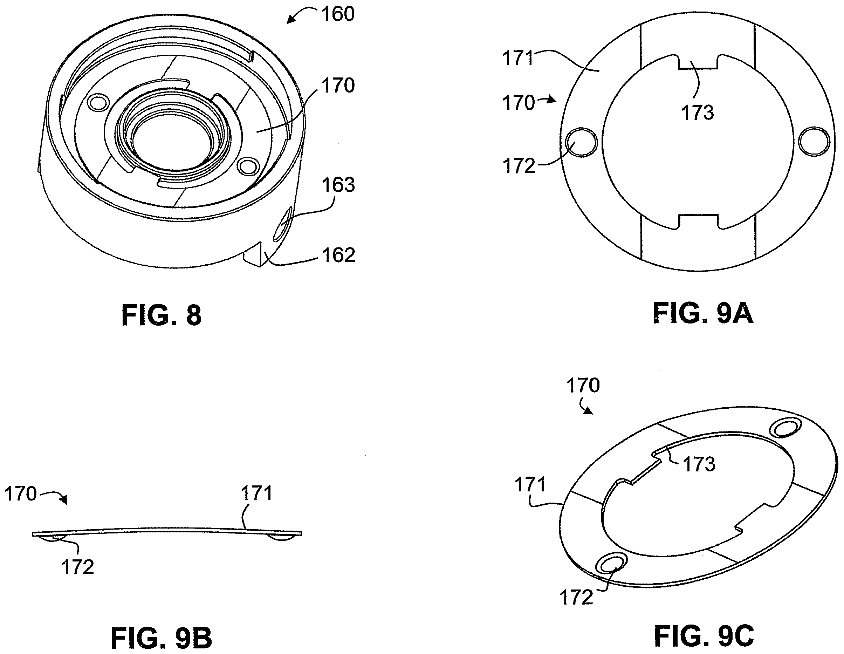

In distaler Richtung grenzt eine Bajonetthülse 160 an die Führungshülse 110 und den Lagerhalter 130 an, die auch in der Fig. 8 dargestellt ist. Sie ist auf dem Lagerhalter 130 in axialer Richtung gehalten und gegenüber diesem verdrehbar. Mit einem nach innen ragenden Ringflansch 161 stützt sie die Einheit aus Führungshülse 110 und Lagerhalter 130 mit darin gehaltener Kupplungshülse 120 in die distale Richtung ab. Die Bajonetthülse 160 weist zwei axial in die distale Richtung ragende und sich diametral gegenüberliegende Arme 162 auf, die radiale Öffnungen 163 aufweisen. In diese Öffnungen sind radial nach aussen ragende Zapfen eingesetzt, die in zwei als Kulissenführungen (Zwangsführungen) wirkenden Führungsschlitzen 155 des Mechanikhalters 150 laufen. Diese Führungsschlitze 155 sind so ausgestaltet, dass die Bajonetthülse 160 bei einer Drehung im Gegenuhrzeigersinn (im Sinne der oben angegebenen Definition, also bei Betrachtung entlang der Längsachse in distaler Richtung) zwangsweise auch axial in die proximale Richtung verschoben wird. Auf diese Weise wird die Einheit aus Führungshülse 110, Lagerhalter 130 und Kupplungshülse 120 in proximaler Richtung verschoben. Umgekehrt bewegt sich diese Einheit bei einer Drehung der Bajonetthülse 160 im Uhrzeigersinn in die distale Richtung. Parallel zu den Führungsschlitzen 155 verläuft ein weiteres Paar Führungsschlitze 156, um radiale Zapfen 36 eines Verriegelungsbereichs 35 der Karpulenhülse 30 aufzunehmen (vgl. Figuren 1 und 3). Beim Einführen der Karpulenhülse 30 in das Gehäuse unterliegt daher auch die Karpulenhülse einer Zwangsführung, so dass die Karpulenhülse 30 eine kombinierte Drehbewegung und Verschiebung ausführt. Die Karpulenhülse 30 ist dabei so ausgestaltet, dass sie bei ihrer Bewegung mit den Armen 162 der Bajonetthülse 160 gekoppelt wird und die Bajonetthülse 160 mitnimmt.

Die Führungsschlitze 155 sind von endlicher Länge und begrenzen die Bewegung der Bajonetthülse zwischen einer distalen und einer proximalen Endstellung. In der Fig. 7 ist die proximale Endstellung dargestellt. Insbesondere erlauben die Führungsschlitze 155 eine Drehung der Bajonetthülse um 90 Grad zwischen diesen Stellungen.

Um die Bajonetthülse in ihren beiden Endstellungen lösbar drehfest gegenüber der Führungshülse 110 und damit gegenüber der Gehäusehülse 20 zu fixieren, ist zwischen der Bajonetthülse 160 und der Führungshülse 110 eine Bajonettfeder 170 angeordnet. Diese ist in den Figuren 9A bis 9C im Detail dargestellt. Die Bajonettfeder 170 weist einen als Federelement wirkenden, im wesentlichen flachen und ringförmigen Grundkörper 171 auf. Aus diesem Grundkörper ragen zwei diametral gegenüberliegende, axial flach vorstehende Ausbuchtungen oder Vorsprünge 172 als Rastelemente oder Rastnocken axial in die distale Richtung vor. Zwei diametral gegenüberliegende flache Zungen 173 ragen nach innen und kommen in entsprechenden flachen Ausnehmungen der Führungshülse 110 zu liegen. Dadurch wird die Bajonettfeder 170 verdrehgesichert an der Führungshülse 110 gehalten. Wie aus der Fig. 9B erkennbar ist, ist der Grundkörper 171 leicht um eine Achse senkrecht zur Längsachse gebogen, und zwar in einer Weise, dass der Krümmungsmittelpunkt auf der selben Seite der Bajonettfeder wie die Vorsprünge 172 liegt (also distal). Hierdurch wird die Bajonettfeder 170 zwischen der Führungshülse 110 und der Bajonetthülse 160 permanent derart vorgespannt, dass die Vorsprünge 172 in distaler Richtung gegen die entsprechende Gegenfläche am Ringflansch 161 gedrückt werden. In dieser Gegenfläche sind vier Vertiefungen vorhanden, die in Abständen von 90 Grad um die Längsachse angeordnet sind. In der proximalen Endstellung kommen die Vorsprünge 172 in einem ersten Paar dieser Vertiefungen zu liegen, während sie in der distalen Endstellung, in der die Bajonetthülse um 90 Grad verdreht ist, in dem zweiten Paar der Vertiefungen aufgenommen werden. Dadurch gibt es also zwei definierte Raststellungen, in denen die Bajonetthülse 160 über die Bajonettfeder 170 mit den Vorsprüngen 172 lösbar mit der Führungshülse 110 verrastet. In beiden Stellungen muss zunächst eine gewisse Kraft überwunden werden, um die Bajonetthülse wieder in Richtung der jeweils anderen Endstellung zu bewegen. Die Vertiefungen können unterschiedlich ausgeprägt sein, z.B. unterschiedlich tief sein, so dass in den beiden Raststellungen eine unterschiedliche Lösekraft erforderlich ist.

In der einen Raststellung wird die Karpulenhülse 30 über ihre Kopplung mit der Bajonetthülse 160 dreh- und verschiebefest an der Führungshülse 110 und damit letztlich am Gehäuse gehalten. In der anderen Raststellung ist die Karpulenhülse 30 vom Gehäuse gelöst. Die Bajonetthülse 160 ist in dieser Stellung wiederum mit der Führungshülse 110 verrastet und dadurch am Gehäuse 20 dreh- und verschiebefest fixiert. Auf diese Weise ist sichergestellt, dass die Karpulenhülse bei Einschieben in die Führungsschlitze 156 die Arme 162 der Bajonetthülse wieder in der korrekten Stellung um die Längsachse "findet" und diese dann nach dem Lösen der Rastverbindung mitnehmen kann.

Im vorliegenden Beispiel sind die Rastelemente als Vorsprünge 172 einstückig mit dem Federelement in Form des Grundkörpers 171 ausgebildet. Stattdessen kann aber auch ein getrenntes Rastelement, z.B. in Form eines starren Rings mit Rastnocken, vorgesehen sein, das vom Federelement in die axiale Richtung gedrückt wird. Das Rastelement kann statt Vorsprüngen auch Vertiefungen aufweisen, die dann mit entsprechenden Vorsprüngen der Gegenfläche zusammenwirken. Im vorliegenden Beispiel ist das Rastelement drehfest zum Gehäuse. Stattdessen kann es auch drehfest zur Bajonetthülse sein. Das Federelement kann zur Erzeugung einer axialen Kraft auch anders ausgestaltet sein. Es sind also vielfache Abwandlungen der hier dargestellten Verriegelung zwischen der Karpulenhülse 30 und dem Gehäuse möglich.

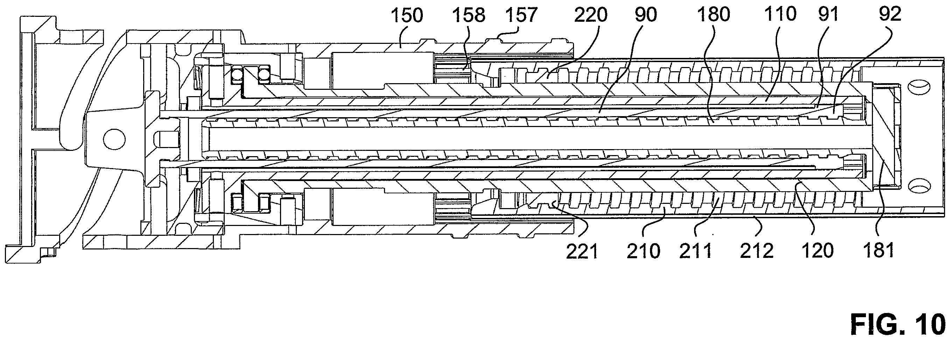

In der Fig. 10 sind die in der Fig. 7 dargestellten Teile der Injektionsvorrichtung zusammen mit weiteren Bauteilen dargestellt. Insbesondere ist nun die Vorschubhülse 90 in die Führungshülse 110 eingeschoben. An ihrem proximalen Ende weist die Vorschubhülse 90 ein kurzes Innengewinde 92 auf, in welchem eine hohle Aussengewindestange 180 geführt ist. Diese ist an ihrem proximalen Ende über einen Querbolzen 181 starr mit der Kupplungshülse 120 verbunden. Ein Vorschub der Vorschubhülse 90 in distaler Richtung erfolgt also, indem die Kupplungshülse 120, die ja drehbar gelagert ist, eine Drehbewegung ausführt. Diese Drehbewegung bewirkt wegen der starren Verbindung zwischen Kupplungshülse 120 und Aussengewindestange 180 eine Drehung auch der Aussengewindestange 180. Die Vorschubhülse 90 läuft mit ihrem Innengewinde 92 auf der Aussengewindestange 180, ähnlich einer Mutter. Die Vorschubhülse 90 ist drehfest gegenüber der Führungshülse 110, da sie über die Führungsnocken 91 in Längsrillen auf der Innenseite der Führungshülse 110 läuft. Auf diese Weise wird die Vorschubhülse 90 bei einer Drehung der Aussengewindestange 180 axial vorgeschoben. Insgesamt wird also eine Drehbewegung der Kupplungshülse 120 in eine axiale Verschiebung der Vorschubhülse 90 umgewandelt.

Wie aus der Fig. 3 erkennbar ist, wird die Vorschubhülse 90 bei dieser Vorschubbewegung durch eine lange, auf Druck belastete Schraubenfeder 190 unterstützt, die im Inneren der Gewindestange 180 angeordnet und auf einer Führungsnadel 200 geführt ist. Die Schraubenfeder 190 drückt eine ringförmige Verdickung 201 nahe dem distalen Ende der Führungsnadel 200 in die distale Richtung gegen den Vorschubflansch 100. Ein axialer Zapfen 202 ragt in ein entsprechendes Sackloch des Vorschubflansches 100 und ist in diesem Sackloch drehbar.

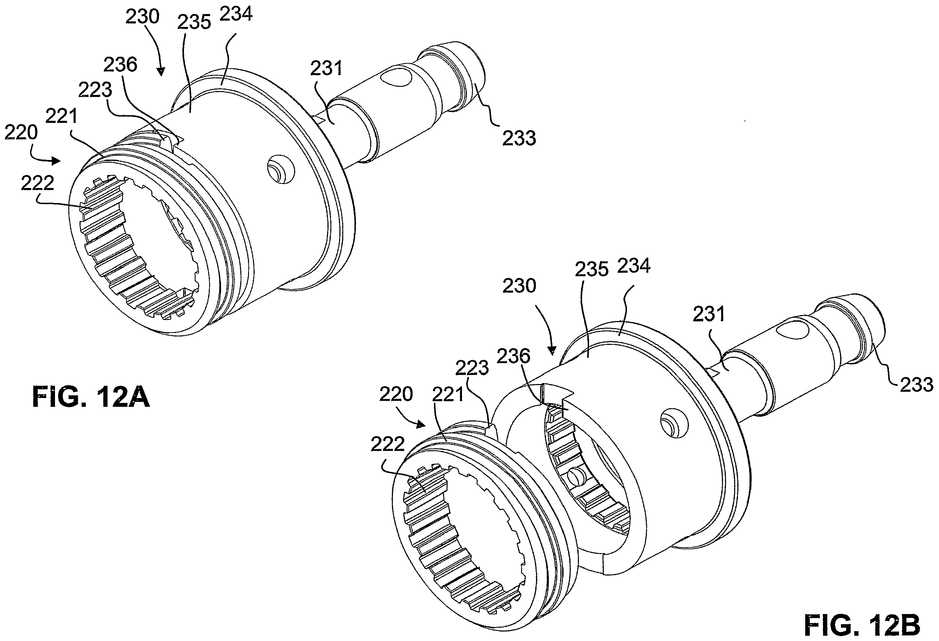

Des weiteren ist in der Fig. 10 in den Mechanikhalter 150 von der proximalen Seite her eine im Wesentlichen zylindrische Übertragungshülse 210 eingeschoben, welche die Kupplungshülse 120 teilweise umgibt. Die Übertragungshülse 210 weist auf der Aussenseite eine Mehrzahl von Längsrippen 212 auf. Der Aussendurchmesser der Übertragungshülse 210 ist dabei so gewählt, dass diese trotz ihrer äusseren Längsrippen frei im Inneren des Mechanikhalters 150 drehbar ist. Auf der Innenseite weist die Übertragungshülse 210 ein Innengewinde 211 auf, in dem ein Dosisbegrenzungsring 220 mit einem entsprechenden Aussengewinde 221 läuft. Im Inneren des Dosisbegrenzungsrings 220 sind in den Figuren 12A und 12B besonders gut erkennbare Längsnuten 222 vorhanden, in welche die Längsrippen 122 der Kupplungshülse 120 (vgl. Fig. 6) eingreifen. Dadurch ist der Dosisbegrenzungsring 220 einerseits drehfest in axialer Richtung auf der Kupplungshülse verschiebbar, andererseits im Innengewinde der Übertragungshülse 210 geführt. Eine Drehung der Kupplungshülse 120 gegenüber der Übertragungshülse 210 führt also zu einer Drehung und axialen Verschiebung des Dosisbegrenzungsrings 220.

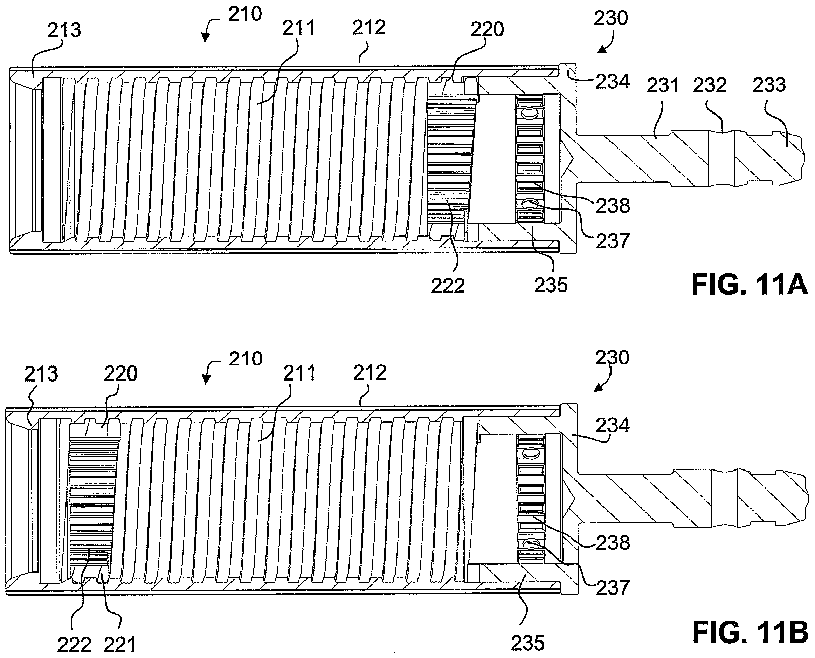

Der axiale Verschiebungsbereich des Dosisbegrenzungsrings ist in distaler wie in proximaler Richtung begrenzt. Dies soll anhand der Figuren 11A, 11B, 12A und 12B erläutert werden.

In den Figuren 11A und 11B ist eine Kupplungswelle 230 mit der Übertragungshülse 210 verbunden. Die Kupplungswelle weist eine Achse 231 mit einer Querbohrung 232 nahe dem proximalen Ende 233 auf. Vom distalen Ende der Achse aus erstreckt sich ein umlaufender Flansch 234 radial nach aussen. Von diesem aus erstreckt sich wiederum ein Ringflansch 235 axial in distaler Richtung. Der Aussendurchmesser des Flansches 234 ist grösser als derjenige des Ringflansches 235, wodurch der Flansch 234 radial über den Ringflansch 235 übersteht und einen Anschlag für die Übertragungshülse 210 bildet. Der Ringflansch 235 ist in die Übertragungshülse 210 eingeschoben, so dass diese mit ihrem proximalen Ende am Flansch 234 ansteht. Der Ringflansch ist über radiale Stifte in der Übertragungshülse 210 gesichert, die in Bohrungen 237 eingeschoben sind. Dadurch sind die Kupplungswelle 230 und die Übertragungshülse 210 drehfest und verschiebegesichert miteinander verbunden. In der Innenfläche des Ringflansches 235 sind mehrere Längsnuten 238 ausgebildet.

Die Figuren 12A und 12B zeigen die Kupplungswelle 230 und den Dosisbegrenzungsring 220 alleine. Am Dosisbegrenzungsring 220 ist ein Radialanschlag 223 ausgebildet, der mit einem entsprechenden Radialanschlag 236 am Ringflansch 235 der Kupplungswelle zusammenwirkt. Unter einem Radialanschlag wird eine Anschlagfläche verstanden, deren Flächennormale im Wesentlichen in die tangentiale Richtung weist, und die dazu ausgebildet ist, mit einer entsprechenden Gegenfläche zusammenzuwirken. Der Radialanschlag wird also primär in tangentialer Richtung (also in Drehrichtung) beansprucht statt in axialer Richtung. Dadurch vermeidet ein Radialanschlag die Gefahr des Verklemmens, die besteht, wenn zwei Teile über eine Schraubenverbindung axial aufeinanderstossen, und die zumal bei geringer Steigung des Schraubengewindes besonders ausgeprägt ist. Der Radialanschlag 236 begrenzt hier die Schraubenbewegung des Dosisbegrenzungsrings 220 in proximaler Richtung. In der Fig. 11A ist der Dosisbegrenzungsring 220 in der resultierenden proximalen Endstellung, in der Fig. 11B dagegen in einer distalen Ausgangsstellung gezeigt.

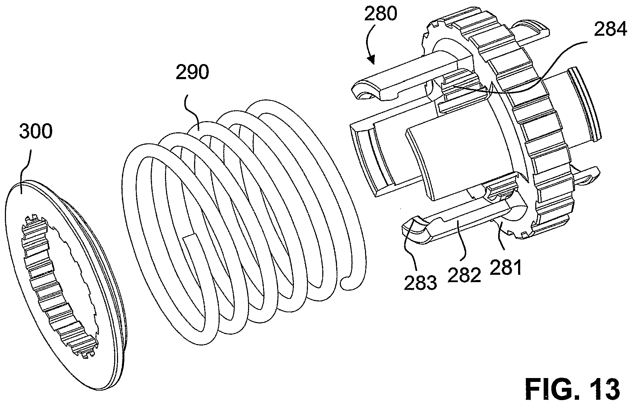

In einen nach innen weisenden, in die distale Richtung abgeschrägten Ringflansch 213 am distalen Ende der Übertragungshülse 210 ist das proximale Ende einer Arretierhülse 280 drehbar eingeklickt. Der besseren Übersichtlichkeit wegen ist die Arretierhülse 280 in der Fig. 10 nicht dargestellt. Sie ist aber in der Fig. 13 gezeigt. Die Arretierhülse umfasst einen ringförmigen Hauptkörper 281, von dem sich vier sich in die distale Richtung erstreckende Arme 282 in die distale Richtung erstrecken. Der Hauptkörper weist an seiner Innenfläche Längsnuten 284 auf, die mit den Längsrippen 122 der Kupplungshülse 120 verzahnt sind. Dadurch ist die Arretierhülse 280 in Längsrichtung relativ zur Kupplungshülse 120 verschiebbar, aber gegenüber dieser verdrehgesichert. Am Ende der Arme 282 sind sich nach innen erstreckende Flanschbereiche 283 vorhanden. Der mögliche Verschiebungsbereich wird in proximaler Richtung durch diese Flanschbereiche begrenzt. Diese stossen in der proximalen Endstellung der Arretierhülse 280, wie sie in der Fig. 3 dargestellt ist, an das distale Ende der Längsrippen 122 der Kupplungshülse 120 an. Auf der äusseren Umfangsfläche des Hauptkörpers 281 sind Längsrippen ausgebildet. Diese Längsrippen greifen in der Stellung der Fig. 3 in die inneren Längsnuten 158 des Mechanikhalters 150 ein. Dadurch ist die Arretierhülse 280 in dieser Stellung axial gegenüber dem Mechanikhalter 150 verschiebbar, aber verdrehgesichert. Letztlich sichert die Arretierhülse 280 in dieser Stellung also die Kupplungshülse 120 gegen eine Drehung im Mechanikhalter 150. Wie weiter unten noch erläutert wird, ist die Arretierhülse 280 jedoch so weit in der distalen Richtung verschiebbar, dass sie ausser Eingriff mit dem Mechanikhalter 150 kommen kann und gegenüber diesem dann zusammen mit der Kupplungshülse 120 drehbar ist.

Die Arretierhülse 280 ist mittels einer Kupplungsfeder 290 in die proximale Richtung vorgespannt. Die Kupplungsfeder hat die Form einer auf Druck belasteten Schraubenfeder, welche die Arme 282 der Arretierhülse 280 umgibt und mit ihrem proximalen Ende an der distalen Stirnseite des Hauptkörpers 281 anliegt. Am distalen Ende der Kupplungsfeder 290 ist diese auf einem Stützring 300 gehalten, der in der distalen Richtung an den Lagerhalter 130 anstösst und an dessen Innenseite Längsnuten ausgebildet sind.

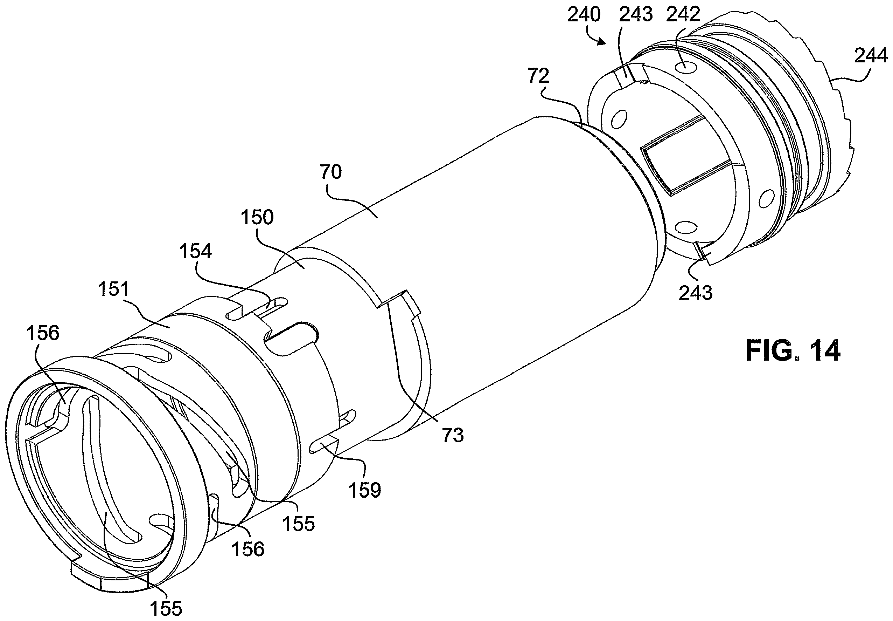

In der Fig. 14 ist nun die Einheit der Fig. 10 mit nochmals weiteren Bauteilen dargestellt. Auf dem Mechanikhalter 150 ist nun die schon zuvor erwähnte Anzeigetrommel 70 gehalten. Ausserdem ist eine Anschlaghülse 240 erkennbar. Wie in der Fig. 2 zu erkennen ist, ist die Anschlaghülse 240 durch Stifte, die in die in der Fig. 14 erkennbaren radialen Löcher 242 ragen, unbeweglich mit der Gehäusehülse 20 verbunden. An ihrem distalen Ende sind Radialanschläge 243 ausgebildet. Ihr proximales Ende ist gezackt ausgebildet, d.h. es sind stirnseitig Zähne 244 für eine noch zu beschreibende Ratschenverbindung ausgebildet.

Die Anzeigetrommel 70 weist ein Innengewinde auf, welches in der Fig. 3 erkennbar ist. Dieses läuft auf dem Aussengewinde 157 des Mechanikhalters, welches besonders gut in den Figuren 7 und 10 erkennbar ist. An ihrem proximalen Ende verjüngt sich die Anzeigetrommel 70 zu einem ringförmigen Bereich 72. An der Innenseite des ringförmigen Bereichs 72 sind Längsnuten ausgebildet. Mit diesen Längsnuten ist die Anzeigetrommel 70 verdrehgesichert, aber in Längsrichtung verschiebbar auf den Längsrippen 212 der Übertragungshülse 210 geführt. Durch die Kombination dieser Längsführung auf der Übertragungshülse und der Gewindeführung auf dem Mechanikhalter führt eine Drehung der Übertragungshülse 210 zu einer kombinierten Drehung und Längsverschiebung der Anzeigetrommel 70. Diese Bewegung ist durch Radialanschläge in beide Richtungen begrenzt. Am proximalen Ende wirkt ein Radialanschlag mit dem Radialanschlag 243 der Anschlaghülse 240 zusammen. Am distalen Ende wirkt ein entsprechender Radialanschlag 73 mit einem Radialanschlag 159 des Mechanikhalters 150 zusammen. Dadurch ist die Schraubenbewegung der Anzeigetrommel 70 in beide Richtungen durch Radialanschläge limitiert.

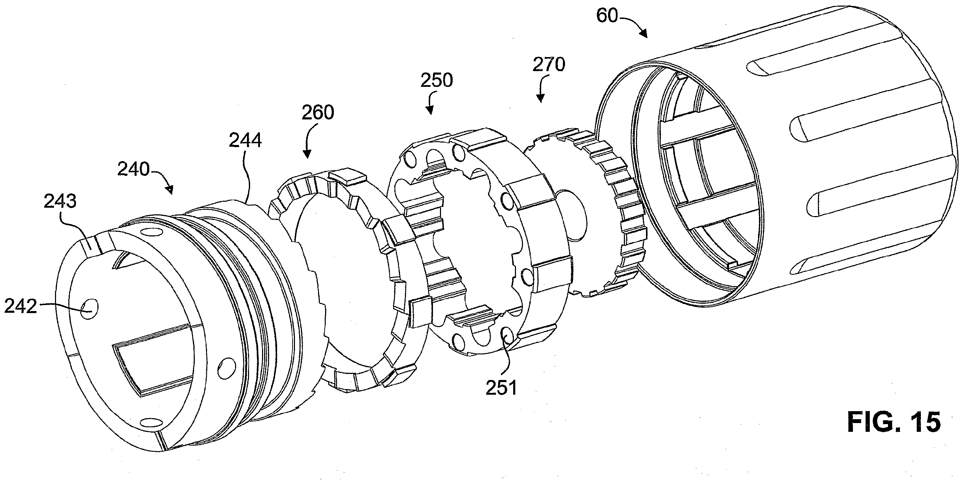

Im Folgenden wird nun der Mechanismus zum Einstellen einer Dosis und zum Auslösen der Verabreichung dieser Dosis anhand der Figuren 3 und 15 erläutert. Am proximalen Ende der Gehäusehülse 20 ist die schon erwähnte Dosierhülse 60 angeordnet. Sie ist mit einem Federring 61 axial verschiebegesichert und drehbar an der Anschlaghülse 240 fixiert. Die Dosierhülse 60 ist über eine Rutschkupplung in Form einer Ratschenverbindung sowohl im Uhrzeigersinn als auch gegen den Uhrzeigersinn um die Längsachse gegen die Gehäusehülse 20 verdrehbar, wobei sie mehrere vordefinierte Raststellungen einnehmen kann. Dieser Mechanismus soll im Folgenden erläutert werden.

Im Inneren der Dosierhülse 60 ist ein Innenring 250 angeordnet und starr mit der Dosierhülse 60 verbunden. Der Innenring 250 weist in seiner radialen Innenfläche eine Mehrzahl von Längsnuten auf. In distaler Richtung vom Innenring 250 ist ein Ratschenring 260 axial verschiebbar, aber drehgesichert in der Dosierhülse 60 gehalten. Der Ratschenring 260 ist auf seiner distalen Stirnseite gezackt ausgebildet, und zwar komplementär zu den Zähnen 244 der gezackten proximalen Stirnseite der Anschlaghülse 240, so dass Zähne des Ratschenrings 260 in Vertiefungen der Stirnseite der Anschlaghülse 240 eingreifen können und umgekehrt. Der Ratschenring 260 ist zwischen der distalen Stirnfläche des Innenrings 250 und der gezackten proximalen Stirnseite der Anschlaghülse 240 um einen gewissen Betrag axial verschiebbar. Der Betrag, um den eine axiale Verschiebung möglich ist, ist dabei mindestens so gross, dass die gezackten Stirnseiten des Ratschenrings 260 und der Anschlaghülse 240 ausser Eingriff gelangen können. Der Ratschenring 260 wird durch eine Federkraft elastisch gegen die Anschlaghülse 240 gedrückt. Hierzu sind im Innenring 250 mehrere axiale Bohrungen 251 in Form von Sacklöchern vorhanden. In mindestens einer dieser Bohrungen, bevorzugt in mindestens zwei Bohrungen in gleichmässigem Abstand entlang des Umfangs des Rings, sind auf Druck belastete Schraubenfedern 252 eingesetzt. Die Schraubenfedern 252 pressen den Ratschenring elastisch gegen die Anschlaghülse.

In der Ruhestellung befindet sich der Ratschenring 260 mit seiner gezackten Stirnseite im Eingriff mit der gezackten Stirnseite der Anschlaghülse 240. Dadurch nehmen der Ratschenring und die mit ihm verbundene Dosierhülse 60 eine von mehreren definierten Winkelstellungen um die Längsachse ein. Bei einer Drehung der Dosierhülse 60 relativ zur Gehäusehülse 20 gleiten die Zähne des Ratschenrings 260 und der Anschlaghülse 240 gegen die axiale Federkraft der Schraubenfedern 252 aufeinander, bis sie ausser Eingriff gelangen und in der nächsten definierten Winkelstellung wieder in Eingriff gelangen. Auf diese Weise entsteht eine durch Drehen mit einem genügenden Drehmoment federnd lösbare Rastverbindung in mehreren vordefinierten Winkelstellungen der Dosierhülse 60 relativ zur Gehäusehülse 20. Dieser Mechanismus kann auch als Doppelrutschkupplung bezeichnet werden.

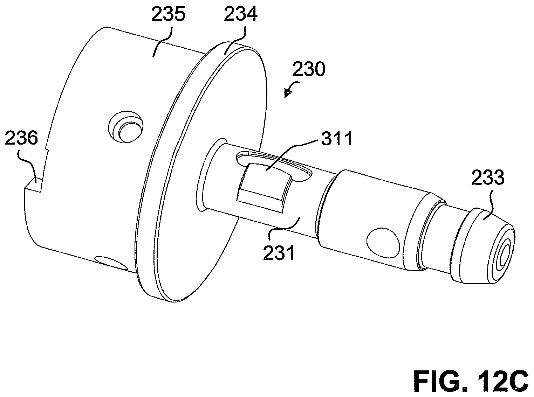

Durch Drehung der Dosierhülse 60 im Uhrzeigersinn lässt sich die Spiralfeder 310 spannen, die in der Fig. 3 angedeutet ist. Die Spiralfeder 310 weist eine Mehrzahl von Federwindungen auf, die um die Längsachse umlaufen und radial zur Längsachse übereinander angeordnet sind. Das innere Ende der Spiralfeder 310 ist an einem in der Fig. 12C besonders gut erkennbaren Federhaltebereich 311 der Kupplungswelle 230 befestigt. Das äussere Ende der Spiralfeder 310 ist an einer Federhülse 320 angebracht, welche drehfest in der Anschlaghülse 240 gehalten ist.

Auf der Kupplungswelle 230 ist eine Kupplungsscheibe 270 montiert und über einen Stift 271 in der Querbohrung 232 der Kupplungswelle 230 gegen Drehung und Verschiebung gesichert. Die Kupplungsscheibe 270 weist an ihrer äusseren Umfangsfläche eine Mehrzahl von Längsrippen auf. In der Stellung der Fig. 3 greifen diese Längsrippen in die dazu komplementären Längsnuten an der Innenseite des Innenrings 250 ein, können aber durch eine axiale Verschiebung ausser Eingriff gebracht werden.

Die Dosierhülse 60 weist eine axiale Durchgangsöffnung auf, in welcher der Druckknopf 80 axial verschiebbar angeordnet ist. Der Druckknopf 80 ist mit einer Mehrzahl von radial federnden Armen 81 drehbar und verschiebegesichert auf dem proximalen Ende 233 der Kupplungswelle 230 aufgeklickt. Er stösst mit seinem distalen Ende gegen eine proximale Stirnfläche der Kupplungsscheibe 270. Im Inneren des Druckknopfs 80 befindet sich eine Schraubenfeder 82, welche mit ihrem proximalen Ende an der inneren Stirnfläche des Druckknopfs anliegt und mit ihrem distalen Ende gegen einen Auflagering 83 drückt. Der Auflagering 83 weist auf seiner äusseren Umfangsfläche Längsrippen auf, die in entsprechenden Längsnuten in der inneren Mantelfäche des Druckknopfes 80 geführt sind. Dadurch ist der Auflagering 83 im Druckknopf 80 drehfest und axial verschiebbar angeordnet. An seiner distalen Stirnseite ist der Auflagering 83 flach gezackt ausgebildet. Die proximale Stirnseite der Kupplungsscheibe 270 ist hierzu komplementär gezackt ausgebildet, so dass der Auflagering 83 mit der Kupplungsscheibe 270 axial verzahnt ist. Beim Ausschütten des Medikaments dreht sich die Kupplungsscheibe 270 gegenüber dem Auflagering 83. Dabei gleiten die gezackten Flächen aufeinander, so dass die Verzahnung abwechselnd in und ausser Eingriff gerät. Dadurch wird ein charakteristisches Klickgeräusch erzeugt, das dem Benutzer anzeigt, dass gerade eine Verabreichung stattfindet. Die Verzahnung von Auflagering 83 und Kupplungsscheibe 270 ist dabei vorzugsweise so ausgestaltet, dass jedes Klicken gerade einer Einheit (oder eines vorbestimmten Vielfachen einer Einheit) des verabreichten Medikaments entspricht.

### Abdichtung

Die gesamte Mechanik für die Einstellung der Dosis und die Ausschüttung ist spritzwassergeschützt in der Gehäusehülse 20 untergebracht. Hierzu sind insgesamt vier Dichtungen D1, D2, D3 und D4 vorhanden. Die Dichtung D1 umfasst einen Dichtring, der zwischen dem Mechanikhalter 150 und der Bajonetthülse 160 dichtend anliegt. Der Mechanikhalter 150 ist unbeweglich und dicht im Gehäuse 2 montiert, während die Bajonetthülse 160 gegenüber dem Mechanikhalter 160 sowohl verschiebbar als auch drehbar ist und dabei über die Dichtung D1 gegenüber dem Gehäuse gedichtet ist. Die Dichtung D2 umfasst einen weiteren, hier flach ausgebildeten Dichtring, der dichtend zwischen der Bajonetthülse 160 und der glatten Aussenseite der Vorschubhülse 90 anliegt. Des weiteren ist der Vorschubflansch 100 dicht auf der Vorschubhülse 90 angeordnet. Damit ist der proximal von der Bajonetthülse 160 liegende Bereich der Injektionsvorrichtung, einschliesslich dem Inneren der Vorschubhülse 90, komplett gegen den distal hiervon liegenden Bereich gedichtet. Falls Flüssigkeiten in diesen distalen Bereich gelangen sollten, z.B. wegen eines Bruchs der Medikamentenkarpule 40, so können diese nicht in die diffizile Mechanik eindringen und diese verschmutzen oder verkleben.

Die anderen beiden Dichtungen befinden sich am proximalen Ende des Injektionsgeräts. Die Dichtung D3 umfasst einen Dichtring, der zwischen der Dosierhülse 60 und der Anschlaghülse 240 dichtend anliegt. Die Anschlaghülse 240 ist unbeweglich und dicht in der Gehäusehülse 20 montiert, während die Dosierhülse 60 gegenüber der Anschlaghülse 240 verdrehbar ist. Die Dichtung D4 umfasst einen weiteren Dichtring, der dichtend zwischen der Dosierhülse 60 und dem Druckknopf 80 anliegt. Zudem ist auf dem Fenster 21 eine transparente Fensterabdeckung 22 flüssigkeitsdicht aufgesetzt. Hierdurch ist die gesamte Mechanik, die nach aussen hin durch die Gehäusehülse 20, die Dosierhülse 60 und den Druckknopf 80 begrenzt wird, auch nach aussen hin komplett gedichtet und so vollständig gegen das Eindringen von Flüssigkeiten geschützt. Auch ein Regenguss oder ein versehentlich vom Benutzer umgeworfenes Wasserglas können dem Injektionsgerät also nichts anhaben.

Insbesondere die Dichtung zur Vorschubhülse hin ist bevorzugt derart ausgebildet, dass sie als Abstreifer wirkt, ähnlich einem Scheibenwischer beim Auto. Hierzu besteht mindestens zur distalen Seite hin vorzugsweise ein möglichst kleiner Kontaktwinkel zwischen den Oberflächen des Dichtelements und der Vorschubhülse. Dieser kann unterhalb von 90 Grad liegen.

Anstelle von konventionellen Dichtungen oder zusätzlich hierzu können die gegeneinander zu dichtenden Teile eine hydrophobe Oberfläche aufweisen, insbesondere aus einem hydrophoben Material ausgebildet oder damit beschichtet sein. Eine hydrophobe Oberfläche verhindert, dass die Teile benetzt werden. Dadurch perlen Wassertropfen ab, und es wird ein Kriechen von Flüssigkeiten durch Spalte zwischen den zu dichtenden Teilen aufgrund von Kapillareffekten wirksam verhindert. Die mit einer hydrophoben Oberfläche versehenen, gegeneinander zu dichtenden Teile können also in einem gewissen Abstand (Spalt) zueinander angeordnet sein, ohne dass die Dichtwirkung verloren geht ("virtuelle Dichtung").

Unter einer hydrophoben Oberfläche wird hier eine Oberfläche verstanden, für die der Kontaktwinkel eines Wassertropfens mindestens 90 Grad beträgt, bevorzugt mindestens 110 Grad. Der Kontaktwinkel ist der Winkel zwischen den Flächennormalen des Wassertropfens und der betreffenden Oberfläche an der Kontaktstelle. Beispiele für Materialien mit hydrophoben Eigenschaften sind PTFE (Polytetrafluorethylen) oder PVDF (Polyvinylidenfluorid). Viele weitere Materialien, insbesondere auch für dünne Beschichtungen im Bereich von wenigen Mikrometern, sind bekannt.

In Versuchen wurden verschiedene Stifte sowie eine Hülse nanotechnologisch mit einer hydrophoben Beschichtung versehen. Es wurden 20 Stifte mit Aussendurchmessern von 10.0 bis 11.9 mm in Abstufungen von 0.1 mm untersucht. Die Stifte wurden zentral in einer Hülse mit 12 mm Innendurchmesser angeordnet, was Spaltdicken von 0.05 mm bis 1.0 mm in Abstufungen von 0.05 mm entspricht. Das Innere der Hülse wurde dann mit Wasser beaufschlagt. Es wurde eine Dichtwirkung bis zu einer Spaltdicke von ca. 0.5 mm beobachtet. Bei gegenseitiger Drehung zwischen Stift und Hülse wurde eine Dichtwirkung bis zu einer Spaltdicke von ca. 0.25 mm beobachtet.

Um die Dichtwirkung zu verbessern, können die Oberflächen mikro- oder nanostrukturiert werden, d.h. mit Strukturen versehen werden, deren Abmessungen im Nanometer- bis Mikrometerbereich liegen. Diese Strukturen können eine Vorzugsrichtung aufweisen, um das Fliessen von Flüssigkeiten auf der Oberfläche in eine Richtung zu hemmen. So können z.B. Schuppen vorgesehen werden.

### Kupplungen

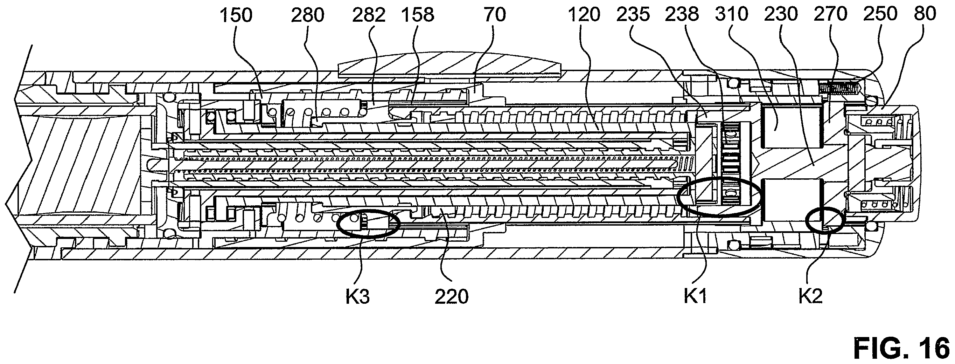

In Folgenden soll nun die Funktionsweise des Injektionsgeräts erläutert werden. Hierzu wird zunächst auf die Fig. 16 verwiesen, in welcher das Injektionsgerät in seiner Ausgangsstellung vor dem ersten Gebrauch dargestellt ist. Die oben erläuterte Mechanik zum Einstellen und Ausschütten einer Dosis verfügt über drei Kupplungen K1, K2, und K3 für die Übertragung von Drehmomenten. Jede dieser Kupplungen kann durch eine axiale Bewegung zweier Bauteile gegeneinander in und ausser Eingriff gebracht werden.

Die Kupplung K1 wird durch die Längsnuten auf der Innenfläche des axialen Flansches 235 der Kupplungswelle 230 als Kupplungseingangsglied im Zusammenspiel mit den Längsrippen 122 auf der Aussenseite der Kupplungshülse 120 (vgl. Fig. 5) als Kupplungsausgangsglied gebildet. In der Stellung der Fig. 16 ist diese Kupplung ausgekuppelt, d.h., die durch die Längsnuten und Längsrippen gebildete Verzahnung ist ausser Eingriff. Die Kupplung K1 lässt sich durch eine axiale Verschiebung der Kupplungswelle 230 in distaler Richtung einkuppeln.

Die Kupplung K2 wird durch die Längsnuten in der radialen Innenfläche des Innenrings 250 als Kupplungseingangsglied im Zusammenspiel mit den Längsrippen auf der radialen Aussenfläche der Kupplungsscheibe 270 als Kupplungsausgangsglied gebildet. In der Stellung der Fig. 16 ist diese Kupplung eingekuppelt, d.h., die durch die Längsnuten und Längsrippen gebildete Verzahnung ist im Eingriff. Die Kupplung K2 lässt sich durch eine axiale Verschiebung der Kupplungsscheibe 270 in distaler Richtung auskuppeln.

Die Kupplung K3 wird durch die Längsrippen auf der Aussenseite des äusseren Ringflansches 282 der Arretierhülse 280 als Kupplungseingangsglied im Zusammenspiel mit den Längsnuten 158 auf der Innenfläche des Mechanikhalters 150 als Kupplungsausgangsglied gebildet. In der Stellung der Fig. 13 ist diese Kupplung eingekuppelt. Sie lässt sich durch eine axiale Verschiebung der Arretierhülse 280 in distaler Richtung auskuppeln.

Alle drei Kupplungen K1, K2 und K3 lassen sich ein- bzw. auskuppeln, indem der Druckknopf 80 genügend weit axial verschoben wird. Dabei wird eine bestimmte Reihenfolge eingehalten. Beim Eindrücken des Druckknopfes 80 werden die Kupplungsscheibe 270 und die mit ihr fest verbundene Kupplungswelle 230 in distaler Richtung verschoben. Dabei kommt zunächst die Kupplung K1 in Eingriff, d.h., die Kupplungswelle wird zur Drehmomentübertragung mit der Kupplungshülse 120 gekoppelt. Gleichzeitig schiebt die Kupplungswelle 230 die Übertragungshülse 210 in die distale Richtung vor. Diese nimmt die Arretierhülse 280 in distaler Richtung mit, wodurch die Kupplungsfeder 290 komprimiert wird. Wenn die Kupplung K1 erstmals in Eingriff gelangt, ist die Arretierhülse 280 noch nicht genügend weit vorgeschoben, um mit ihrem äusseren Ringflansch 282 ausser Eingriff mit dem Mechanikhalter 250 zu gelangen. Die Kupplung K3 ist also zunächst weiterhin eingekuppelt. Dasselbe gilt für die Kupplung K2: Die Kupplungsscheibe 270 ist zunächst noch immer im Eingriff mit dem Innenring 250. Zunächst sind nun also alle drei Kupplungen eingekuppelt. Wenn der Druckknopf 80 weiter eingeschoben wird, gerät als nächstes die Kupplung K2 ausser Eingriff. Bei einem noch weiteren Einschieben gerät schliesslich auch die Kupplung K3 ausser Eingriff. Es wird also die folgende Reihenfolge eingehalten:
- Ausgangszustand: K1 ausgekuppelt, K2 und K3 eingekuppelt.
- Eindrücken des Druckknopfs 80: K1 kuppelt ein, danach kuppelt K2 aus, danach kuppelt K3 aus.

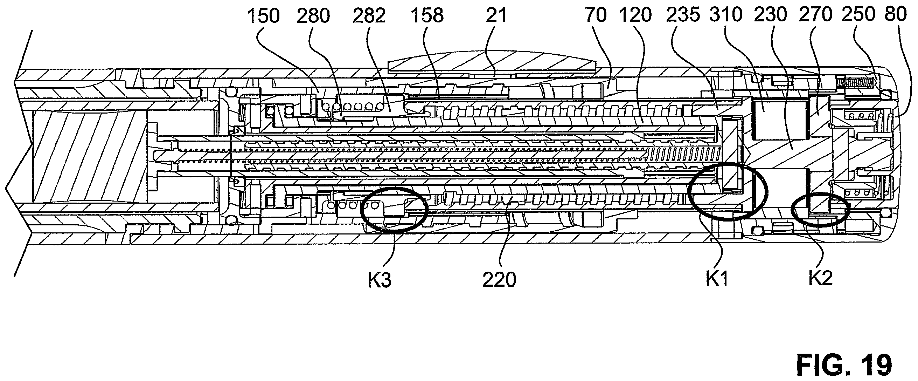

Die später noch näher beschriebene Fig. 19 zeigt das Injektionsgerät bei vollständig eingedrücktem Druckknopf 80. Die Kupplung K1 ist nun eingekuppelt, während die Kupplungen K2 und K3 ausgekuppelt sind.

Bei Loslassen des Druckknopfs 80 läuft das Ineinandergreifen der Kupplungen in umgekehrter Reihenfolge ab. Dabei drückt die Kupplungsfeder 290 die Arretierhülse 280, die Übertragungshülse 210, die Kupplungswelle 230, die Kupplungsscheibe 270 und den Druckknopf 80 in die distale Ausgangsstellung zurück.

Die Kupplungen K1, K2 und K3 sowie die Ratschenverbindung ermöglichen die gezielte Übertragung von Drehmomenten zwischen fünf funktional selbstständigen Einheiten. Eine erste Einheit wird durch die Gehäusehülse 20, den Mechanikhalter 150, die Anschlaghülse 240 und den Federring 320 gebildet. Diese Einheit kann funktional als Halteeinrichtung oder als ein Gehäuse in einem erweiterten Sinn aufgefasst werden. Sie stellt das stationäre Bezugssystem für alle Bewegungen dar.

Eine zweite Einheit wird durch die Dosierhülse 60, den Innenring 250 und den Ratschenring 260 gebildet. Sie kann funktional als drehbare Dosiereinrichtung aufgefasst werden. Diese Dosiereinrichtung wird über die Ratschenverbindung am Gehäuse lösbar, aber bis zu einem gewissen Wert drehmomentenfest gehalten.

Eine dritte Einheit wird durch die Kupplungsscheibe 270, die Kupplungswelle 230 und die Übertragungshülse 210 gebildet, die ja alle starr miteinander verbunden sind, sowie durch die damit verbundene Spiralfeder 310, die als das eigentliche Antriebselement wirkt. Diese Einheit kann als Antriebseinrichtung aufgefasst werden. Die Drehbewegung der Antriebseinrichtung ist durch zwei Begrenzungselemente limitiert, die beide an der Übertragungseinrichtung geführt sind. Das erste Begrenzungselement wird durch die Anzeigetrommel 70 gebildet, welche den Bewegungsbereich der Antriebseinrichtung in beide Richtungen, einer Dosierrichtung und einer Korrektur- und Ausschüttrichtung, limitiert. Das zweite Begrenzungselement wird durch den Dosisbegrenzungsring 220 gebildet, welcher den Bewegungsbereich der Antriebsseinrichtung zumindest in einer Richtung, der Dosierrichtung, unabhängig vom ersten Begrenzungselement begrenzt. Die Antriebseinrichtung ist durch die Kupplung K2 lösbar drehfest mit der Dosiereinrichtung koppelbar, was es ermöglicht, das Antriebselement in Form der Spiralfeder 310 zu spannen.

Eine vierte Einheit umfasst die Kupplungshülse 120 und die Gewindestange 180, die eine starre Einheit bilden, die Elemente, auf denen diese Teile gelagert sind, nämlich die Führungshülse 110, den Lagerhalter 130 und die Kugellagerringe 140, sowie die Vorschubhülse 90. Diese Einheit stellt eine Fördereinrichtung dar, die eine Drehbewegung eines Eingangsglieds in Form der Kupplungshülse 120 in einen Vorschub des Förderelements in Form der Vorschubhülse 90 umwandelt. Ihr Eingangsglied ist durch die Kupplung K1 lösbar drehfest mit der Antriebseinrichtung koppelbar. Zudem ist es über die Kupplung K3 lösbar drehfest mit der Halteeinrichtung (d.h. dem Gehäuse) koppelbar.

Des weiteren ist eine Auslöseeinrichtung vorhanden, die primär den Druckknopf 80 umfasst und zur Bedienung der Kupplungen K1 bis K3 dient.

### Funktionsweise

Das Injektionsgerät wird wie folgt bedient. Ausgehend von der Ausgangsstellung der Fig. 16 wird zunächst eine zu verabreichende Dosis eingestellt. Hierzu wird die Dosierhülse 60 im Uhrzeigersinn gedreht. Die Dosierhülse nimmt dabei über die Kupplung K2 die Kupplungsscheibe 270 und die Kupplungswelle 230 mit. Hierdurch wird die Spiralfeder 310 aufgezogen. Das hierdurch entstehende Drehmoment wird durch die Ratschenverbindung zwischen dem mitdrehenden Ratschenring 260 und der stationären Anschlaghülse 240 gehalten. Durch die Drehung der Kupplungswelle 230 werden auch die Übertragungshülse 210 und die darauf geführte Anzeigetrommel 70 mitgedreht. Die Anzeigetrommel 70 wird wegen ihrer Gewindeführung am Mechanikhalter 150 zudem axial in proximaler Richtung verschoben, führt also insgesamt eine Schraubenbewegung in proximaler Richtung aus. Hierdurch wandern Markierungen auf der Oberfläche der Anzeigetrommel 70 unter dem Fenster 21 durch und zeigen die aktuell eingestellte Dosis an. Des weiteren wird der Dosisbegrenzungsring 220 aufgrund seines Gewindeeingriffs mit dem Inneren der Übertragungshülse 210 und aufgrund seiner drehgesicherten Anordnung auf der Kupplungshülse 120 in proximaler Richtung verschoben.

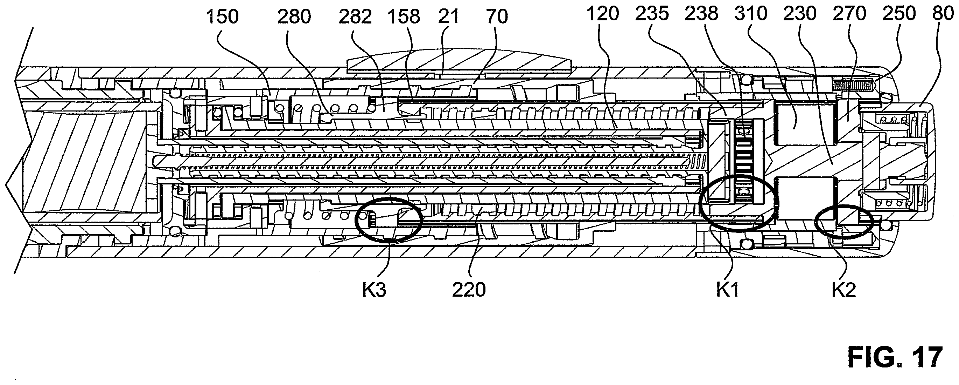

Die Fig. 17 zeigt das Injektionsgerät, nachdem die halbe maximale Einzeldosis eingestellt wurde. Die Anzeigetrommel ist den halben Weg zwischen ihrer distalen und ihrer proximalen Endstellung nach hinten gewandert. Ausserdem ist der Dosisbegrenzungsring 220 um einen Betrag in die proximale Richtung gewandert, der proportional zur eingestellten Einzeldosis ist.

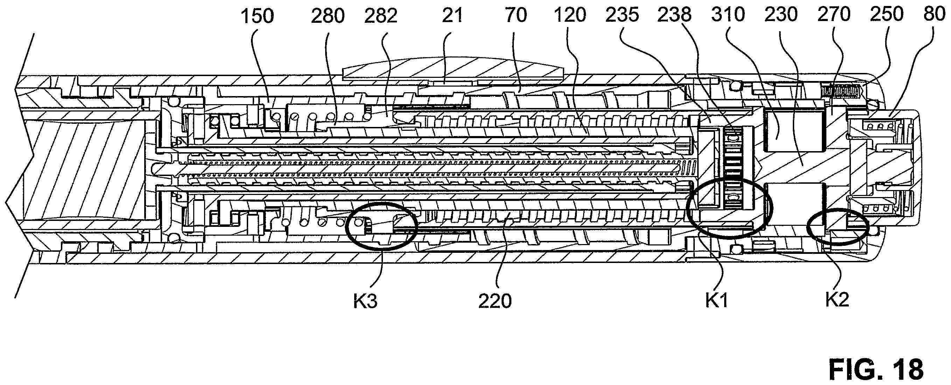

Die Drehung der Dosierhülse 60 im Uhrzeigersinn wird einerseits durch den maximalen Bewegungsbereich der Anzeigetrommel 70, andererseits durch den maximalen Bewegungsbereich des Dosisbegrenzungsrings 220 limitiert. Die Anzeigetrommel 70 stösst nach einer vorbestimmten Zahl von Umdrehungen der Dosierhülse 60 mit ihrem proximalen Radialanschlag gegen die Anschlaghülse 240, sofern die Drehung der Dosierhülse 60 nicht schon zuvor durch den Dosisbegrenzungsring 220 limitiert wurde, wie unten näher beschrieben wird. Dadurch ist kein Weiterdrehen der Dosierhülse 60 mehr möglich. Diese Stellung entspricht der maximal einstellbaren Einzeldosis. Diese Situation ist in der Fig. 18 dargestellt.

Soll die eingestellte Dosis korrigiert, also verringert, werden, so lässt sich die Dosierhülse 60 gegen die Kraft der Ratschenverbindung im Gegenuhrzeigersinn zurückdrehen. Da die Ratschenverbindung in diese Richtung zusätzlich auch das Drehmoment der Spiralfeder 310 aufzunehmen hat, ist die Ratschenverbindung asymmetrisch ausgestaltet: Die stirnseitige Verzahnung weist auf derjenigen Seite, die durch ein Drehmoment belastet ist, das im Gegenuhrzeigersinn auf die Dosierhülse wirkt, einen grösseren Steigungswinkel auf als auf derjenigen Seite, die bei einem Drehmoment im Uhrzeigersinn belastet wird (vgl. die Ausgestaltung der Zähne 244 in Fig. 14). Unter dem Steigungswinkel wird dabei der Absolutbetrag des Winkels zwischen der jeweiligen Flanke eines Zahnes auf der Stirnseite des Ratschenrings 260 bzw. auf der Stirnseite der Anschlaghülse 240 und einer Querschnittsfläche durch den Injektor verstanden.

Die Ausschüttung der so eingestellten Dosis erfolgt, indem der Druckknopf 80 eingedrückt wird. Hierdurch wird zunächst die Kupplung K1 eingekuppelt. Dadurch entsteht eine drehfeste Verbindung zwischen der Kupplungswelle 230 einerseits und der Kupplungshülse 120 sowie der damit starr verbundenen Gewindestange 180 andererseits. Nun sind alle drei Kupplungen K1, K2 und K3 eingekuppelt. Beim weiteren Eindrücken des Druckknopfs 80 kuppelt die Kupplung K2 aus. Dadurch wird die drehfeste Verbindung zwischen der Dosierhülse 60 einerseits und der Kupplungswelle 230 mit der nun angekoppelten Kupplungshülse 120 und Gewindestange 180 andererseits aufgehoben. Dies führt dazu, dass die Ratschenverbindung das Drehmoment der Spiralfeder 310 nicht mehr aufnimmt. Das System ist aber immer noch drehfest über die Kupplung K3 im Mechanikhalter 150 und damit in der Gehäusehülse 20 gehalten. Wenn der Druckknopf 80 nun noch weiter eingedrückt wird, kuppelt auch die Kupplung K3 aus. In diesem Moment wird das Drehmoment der Spiralfeder 310 frei und wirkt über die Kupplungswelle 230 und die Kupplungshülse 120 auf die Gewindestange 190. Hierdurch werden diese Teile in eine Drehung im Gegenuhrzeigersinn versetzt. Durch ihren Gewindeeingriff mit der Gewindestange 190 erfährt die Vorschubhülse 90 eine axiale Verschiebung in distaler Richtung. Über den Vorschubflansch 100 schiebt die Vorschubhülse den Stopfen 41 in der Karpule 40 vor. Auf diese Weise wird das Medikament ausgeschüttet.

Während der Ausschüttung wirken erhebliche Axialkräfte auf die Vorschubhülse 90: Das Drehmoment der Spiralfeder 310 wird in eine Kraft in Vorschubrichtung umgewandelt, die den Stopfen 41 in der Karpule 40 vortreibt. Diese Kräfte werden durch die Kugellager zwischen der Kupplungshülse 120 einerseits und der Führungshülse 110 und dem Lagerhalter 130 andererseits reibungsarm aufgenommen, so dass reibungsbedingte Gegenkräfte, die das antreibende Drehmoment mindern könnten, minimiert werden.

Bei der Ausschüttung wird die Anzeigetrommel 70 durch die Drehung der Übertragungshülse 210 im Gegenuhrzeigersinn wiederum mitgenommen und dadurch wegen ihres Gewindeeingriffs mit dem stationären Mechanikhalter 150 wieder in distaler Richtung bewegt, bis sie wieder ihre distale Ausgangsstellung einnimmt. In dieser Stellung wird sie durch einen Radialanschlag am Weiterdrehen gehindert, wodurch die Ausschüttung beendet wird. Nach Ende der Ausschüttung zeigt die Anzeigetrommel also wieder die Dosis "Null" an.

Die Ausschüttung kann jederzeit unterbrochen werden, indem der Druckknopf 80 losgelassen wird. Dadurch kuppeln die Kupplungen K3 und K2 wieder ein, und die Kupplung K1 kuppelt wieder aus. Die Anzeigetrommel 70 zeigt die verbleibende Restdosis an, die noch ausgeschüttet wird, wenn der Druckknopf wieder gedrückt wird und dadurch die Ausschüttung fortgesetzt wird.

Der Dosisbegrenzungsring 220 behält während der Ausschüttung seine axiale Lage bei, da die Übertragungshülse 210 und die Kupplungshülse 120, zwischen denen sich der Dosisbegrenzungsring 220 befindet, synchron drehen.

Nach dem Ende der Ausschüttung steht die Injektionsvorrichtung für den nächsten Injektionsvorgang bereit. Gegenüber der Fig. 16 haben jedoch zwei Bauteile ihre Position verändert: Einerseits ist die Vorschubhülse 90 entsprechend der ausgeschütteten Dosis in die distale Richtung gewandert. Andererseits ist der Dosisbegrenzungsring 220 ebenfalls um einen dazu proportionalen Betrag in proximaler Richtung gewandert. Hiervon abgesehen, entspricht der Zustand nach dem Ende der Injektion dem Ausgangszustand der Fig. 16. Mit jeder weiteren Injektion wandert also die Vorschubhülse 90 weiter in die distale Richtung, während der Dosisbegrenzungsring 220 in die proximale Richtung wandert. Dies ist in der Fig. 19 illustriert, welche die Injektionsvorrichtung darstellt, nachdem zunächst eine erste Dosis verabreicht wurde, die der halben maximalen Einzeldosis entspricht, und anschliessend mit der Dosierhülse erneut eine Dosis eingestellt wurde, die wiederum der halben maximalen Einzeldosis entspricht. Die Anzeigetrommel zeigt nun erneut, wie in der Fig. 15, die halbe maximale Einzeldosis an, während der Dosisbegrenzungsring 220 nun eine Position in der Übertragungshülse 210 einnimmt, die der Summe der bisher eingestellten Dosen, hier also zweimal der halben maximalen Einzeldosis, entspricht.

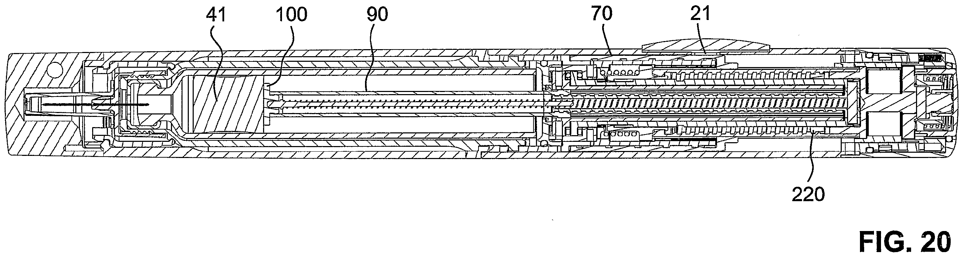

Der maximale axiale Weg, um den der Dosisbegrenzungsring 220 in proximaler Richtung in der Übertragungshülse wandern kann, entspricht gerade dem Inhalt einer vollständig gefüllten Karpule. Sobald die Summe der eingestellten Dosen an der Dosierhülse dem Karpuleninhalt entspricht, erreicht der Dosisbegrenzungsring 220 seine proximale Endstellung und stösst mit seinem Radialanschlag am axialen Ringflansch 235 der Kupplungswelle 230 an, wie dies in der Fig. 12A dargestellt ist. Dadurch wird die Dosierhülse 60 an einem weiteren Verdrehen im Uhrzeigersinn gehindert. Es kann also keine grössere Dosis eingestellt werden als diejenige Dosis, die der verbleibenden Restmenge des Medikaments in der Karpule entspricht. Die Fig. 20 zeigt diese Situation, in welcher kein Aufdosieren mehr möglich ist, obwohl sich die Anzeigetrommel in der distalen Ausgangsstellung, also der Nullstellung, befindet. Entsprechend hat die Vorschubhülse 90 ihre distale Endstellung erreicht, ist also maximal ausgefahren.

### Karpulenwechsel

Um die Karpule auszuwechseln, wird die Karpulenhülse 30 gegen den elastischen Widerstand der Bajonettfeder 170 aus dem Mechanikhalter 150 gelöst und durch den entsprechenden Führungsschlitz 156 im Mechanikhalter geführt herausgedreht. Hierdurch wird zwangsweise die Bajonetthülse 160 entlang ihrem eigenen, parallelen Führungsschlitz 155 ebenfalls verdreht und gleichzeitig in distaler Richtung verschoben. Hierdurch wird die Führungshülse 110 in die distale Richtung gezogen. Hierdurch wandern auch alle damit axial verbundenen beweglichen Teile, insbesondere die Kupplungshülse 120, die Gewindestange 190, die Arretierhülse 280, die Übertragungshülse 210, die Kupplungswelle 230, die Kupplungsscheibe 270 und der Druckknopf 80 in die distale Richtung. Insbesondere wird der Druckknopf 80 also in die Dosierhülse 60 eingezogen und zeigt so an, dass das Injektionsgerät nicht betriebsbereit ist.

Durch diese axiale Verschiebung der verschiedenen Teile der Mechanik kommen die Kupplungen K2 und K3 ausser Eingriff, während K1 ohnehin schon ausser Eingriff war. Sollte vor dem Karpulenwechsel noch eine Dosis eingestellt, aber nicht verabreicht worden sein, so versetzt die dementsprechend aufgezogene Spiralfeder 310 nun die Kupplungswelle 230 und die damit verbundene Übertragungshülse 210 in eine Drehung im Gegenuhrzeigersinn, bis die Anzeigetrommel 70 ihre distale Endstellung erreicht hat und durch ihren Radialanschlag am Mechanikhalter 150 ein weiteres Zurückdrehen verhindert. Auf diese Weise wird die Anzeigetrommel 70 in ihre distale Ausgangsstellung, die Nullstellung, zurückgebracht. Es erfolgt also eine automatische Rückstellung der Dosisanzeige auf Null.

Falls sich vor dem Karpulenwechsel noch eine Restmenge des Medikaments in der Karpule 40 befunden haben sollte, so war die Vorschubhülse 90 vor dem Karpulenwechsel noch nicht maximal ausgefahren, hatte also noch nicht ihre distale Endstellung erreicht. Beim Abnehmen der Karpulenhülse 30 drückt die Schraubenfeder 190 nun die Führungsnadel 200, den Vorschubflansch 100 und die Vorschubhülse 90 in die distale Richtung. Hierdurch wird die Gewindestange 180 über ihre Schraubenverbindung mit dem Inneren der Vorschubhülse 90 in Drehung versetzt. Dabei nimmt die Gewindestange 180 insbesondere die Kupplungshülse 120 und den Dosisbegrenzungsring 220 mit. Der Dosisbegrenzungsring 220 wird bei dieser Drehung durch seinen Gewindeeingriff mit der Übertragungshülse 210 in seine proximale Endstellung verschoben. Sobald der Dosisbegrenzungsring 220 diese Ausgangsstellung erreicht hat, verhindert er eine weitere Drehung der Kupplungshülse 120 und der Gewindestange 180, so dass kein weiteres Ausfahren der Vorschubhülse 90 mehr möglich ist. Die Vorschubhülse 90 hat dadurch nun ihre distale Endstellung erreicht. Diese Situation ist in der Fig. 1 dargestellt. Insgesamt befindet sich nun also die Anzeigetrommel 70 in der Nullstellung, der Dosisbegrenzungsring 220 in der proximalen Endstellung und die Vorschubhülse 90 in ihrer distalen Endstellung.

Nun wird eine neue Karpule 40 in die Karpulenhülse 30 eingeschoben, und die Karpulenhülse 30 mit der darin aufgenommenen Karpule 40 wird axial in die proximale Richtung gegen die Gehäusehülse 20 geführt. Dabei drückt zunächst der Stopfen 41 der Karpule den Vorschubflansch 100 und die Vorschubhülse 90 gegen die Kraft der Schraubenfeder 190 in die proximale Richtung. Hierdurch wird wiederum die Gewindestange 180 in Drehung versetzt. Die Gewindestange nimmt erneut die Kupplungshülse 120 und den Dosisbegrenzungsring 220 mit. Dadurch wird der Dosisbegrenzungsring aufgrund seines Gewindeeingriffs mit der Übertragungshülse 210 in die distale Richtung, d.h. in Richtung seiner Ausgangsstellung verschoben. Der Betrag, um den er in diese Richtung verschoben wird, entspricht gerade der in der Karpule 40 vorhandenen Dosis. Bei einer vollständig gefüllten Karpule wandert der Dosisbegrenzungsring 220 in seine distale Ausgangsstellung. Die Karpulenhülse 30 wird dann in den Mechanikhalter 150 eingeschoben, wobei die radialen Zapfen 36 der Karpulenhülse 30 wieder in die Führungsschlitze 156 im Mechanikhalter 150 eingreifen (vgl. Figuren 1 und 3). Durch die Zwangsführung der Karpulenhülse 30 beim Einsetzen in den Mechanikhalter 150 wird wiederum die Bajonetthülse 160 gezwungen, der Bewegung der Karpulenhülse 30 in den entsprechenden Führungsschlitzen zu folgen. Die Bajonetthülse 160 wird dadurch zurück in ihre proximale Endstellung gebracht, in welcher sie durch die Bajonettscheibe 170 lösbar verriegelt wird (vgl. Figuren 8 bis 10). Das Injektionsgerät befindet sich nun wieder in der Ausgangsstellung der Fig. 16 und steht nach dem Aufschrauben eines neuen Nadelhalters 31 für eine neue Abfolge von Verabreichungen zur Verfügung.

### Zweite Ausführungsform

In der Fig. 21 ist als Variante eine zweite Ausführungsform des erfindungsgemässen Injektionsgeräts dargestellt. Die Funktionsweise ist im Wesentlichen die selbe wie bei der ersten Ausführungsform. Gleich wirkende Teile sind daher mit den gleichen Bezugsziffern bezeichnet wie in der ersten Ausführungsform. Im Folgenden soll lediglich auf die wesentlichen Unterschiede eingegangen werden.

In der zweiten Ausführungsform entfällt die Anschlaghülse 240. Ihre Funktion wird von der entsprechend verlängerten Gehäusehülse 20 übernommen.

Die Antriebseinrichtung, die in der ersten Ausführungsform, abgesehen von der Spiralfeder 310, aus der Kupplungsscheibe 270, Kupplungswelle 230 und Übertragungshülse 210 gebildet wird, ist hier durch andere Teile gebildet, nämlich durch eine Verbindungswelle 400 (mit daran einstückig ausgebildeter Kupplungsscheibe 401), eine am proximalen Ende geschlossene erste Übertragungshülse 410 und eine daran distal anschliessende zweite Übertragungshülse 420. Diese drei Teile sind wiederum starr miteinander verbunden.

Während in der ersten Ausführungsform die Anzeigetrommel nicht nur zur Anzeige der eingestellten Dosis diente, auch zur Begrenzung der maximal einstellbaren Einzeldosis in Dosierrichtung und zur Begrenzung der Bewegung in Ausschüttrichtung, wird die letztere Funktion in der zweiten Ausführungsform durch einen zweiten Dosisbegrenzungsring 430 übernommen. Dieser ist drehfest, aber axial verschiebbar in der Gehäusehülse 20 geführt. Mit einem Innengewinde läuft er auf einem entsprechenden Aussengewinde der ersten Übertragungshülse 410. Seine axiale Bewegung ist durch zwei Radialanschläge zwischen einer distalen Ausgangsstellung, welcher der Nullstellung entspricht, und einer proximalen Endstellung, welcher der maximal einstellbaren Dosis entspricht, begrenzt. Auf diese Weise übernimmt er die Anschlagsfunktionen der Anzeigetrommel gemäss der ersten Ausführungsform.

Die Anzeigetrommel 70 ist in der zweiten Ausführungsform über eine starr mit ihr verbundene Mitnahmehülse 440 axial verschiebbar und drehfest auf der zweiten Übertragungshülse 420 geführt. Ihre Funktionsweise ist ansonsten gleich wie bei der ersten Ausführungsform.

Von diesen Unterschieden abgesehen, sind Aufbau und Funktionsweise des Injektionsgeräts im Wesentlichen gleich wie bei der ersten Ausführungsform.

### Bezugszeichenliste

- R: Medikamentenreservoir
- D1: Dichtung
- D2: Dichtung
- D3: Dichtung
- D4: Dichtung
- K1: erste Kupplung
- K2: zweite Kupplung
- K3: dritte Kupplung
- 10: Schutzhülse
- 11: Haltering
- 12: Rastarm
- 20: Gehäusehülse
- 21: Fenster
- 22: Fensterabdeckung
- 30: Karpulenhülse
- 31: Nadelhalter
- 32: Hohlnadel
- 33: Nadelschutzhülle
- 34: Sichtfenster
- 35: Verriegelungsbereich
- 36: Zapfen
- 40: Karpule
- 41: Stopfen
- 50: Schutzkappe
- 60: Dosierhülse
- 61: Federring
- 70: Anzeigetrommel
- 72: ringförmiger Bereich
- 73: Radialanschlag
- 80: Druckknopf
- 81: Arm
- 82: Schraubenfeder
- 83: Auflagering
- 90: Vorschubhülse
- 91: Führungsnocken
- 92: Innengewinde
- 100: Vorschubflansch
- 110: Führungshülse
- 111: Flansch
- 112: Bohrung
- 120: Kupplungshülse
- 121: Hülsenkörper
- 122: Längsrippe
- 123: Verdickung
- 124: Flansch
- 130: Lagerhalter
- 132: Schulter
- 140: Kugellagerring
- 141: Lagerkugel
- 150: Mechanikhalter
- 151: distaler Abschnitt
- 152: proximaler Abschnitt
- 153: Stufe
- 154: Längsschlitz
- 155: Führungsschlitz
- 156: Führungsschlitz
- 157: Aussengewinde
- 158: Längsnut
- 159: Radialanschlag
- 160: Bajonetthülse
- 161: Ringflansch
- 162: Arm
- 163: Öffnung
- 170: Bajonettfeder
- 171: Grundkörper
- 172: Vorsprung
- 173: Zunge
- 180: Aussengewindestange
- 181: Querbolzen
- 190: Schraubenfeder
- 200: Führungsnadel
- 201: Verdickung
- 202: Zapfen
- 210: Übertragungshülse
- 211: Innengewinde
- 212: Längsrippe
- 213: Ringflansch
- 220: Dosisbegrenzungsring
- 221: Aussengewinde
- 222: Längsnut
- 223: Radialanschlag
- 230: Kupplungswelle
- 231: Achse
- 232: Querbohrung
- 233: proximales Ende
- 234: Flansch
- 235: Ringflansch
- 236: Radialanschlag
- 237: Bohrung
- 238: Längsnut
- 240: Anschlaghülse
- 242: Loch
- 243: Radialanschlag
- 244: Zahn
- 250: Innenring
- 251: Bohrung
- 252: Schraubenfeder
- 260: Ratschenring
- 270: Kupplungsscheibe
- 271: Stift
- 280: Arretierhülse
- 281: Hauptkörper
- 282: Arm
- 283: Flanschbereich
- 284: Längsnut
- 290: Kupplungsfeder
- 300: Stützring
- 310: Spiralfeder
- 311: Federhaltebereich
- 320: Federhülse
- 400: Verbindungswelle
- 401: Kupplungsscheibe
- 410: erste Übertragungshülse
- 420: zweite Übertragungshülse
- 430: zweiter Dosisbegrenzungsring
- 440: Mitnahmehülse

## Patentansprüche

1. Vorrichtung zur Verabreichung eines fluiden Produkts,
- mit einem Gehäuse (20),
- mit einer Fördereinrichtung (90, 110, 120, 130, 140, 180) zum Fördern des Produkts aus einem Reservoir (R, 40), wobei die Fördereinrichtung ein drehbares Eingangslied (120) und ein entlang einer Vorschubachse bewegbares Förderelement (90) umfasst, welches durch eine Drehung des Eingangslieds (120) antreibbar ist,
- mit einer Antriebseinrichtung (210, 230, 270, 310) zum Erzeugen einer Antriebsdrehbewegung um die Vorschubachse relativ zum Gehäuse (20), wobei die Antriebseinrichtung (210, 230, 270, 310) durch eine Spanndrehbewegung spannbar ist,
- mit einer drehbaren Dosiereinrichtung (60) zum Einstellen einer zu verabreichenden Dosis des Produkts und Spannen der Antriebseinrichtung (210, 230, 270, 310); und
- mit einer Auslöseeinrichtung (80), die zum Auslösen einer Verabreichung von einer Ruhestellung in eine Auslösestellung in distale Richtung gegenüber dem Gehäuse (20) und der Dosiereinrichtung (60) bewegbar ist,
- wobei zwischen der Antriebseinrichtung (210, 230, 270, 310) und dem Eingangsglied (120) der Fördereinrichtung (90, 110, 120, 130, 140, 180) eine erste Kupplung (K1) ausgebildet ist, wobei die Antriebseinrichtung und das Eingangsglied der Fördereinrichtung in einer eingekuppelten Stellung drehfest miteinander verbunden sind und in einer ausgekuppelten Stellung voneinander gelöst sind,
- wobei zwischen der Dosiereinrichtung (60) und der Antriebseinrichtung (219, 230, 270, 310) eine zweite Kupplung (K2) ausgebildet ist, wobei die Dosiereinrichtung und die Antriebseinrichtung in einer eingekuppelten Stellung drehfest miteinander verbunden sind und in einer ausgekuppelten Stellung voneinander gelöst sind,
- wobei in der Ruhestellung der Auslöseeinrichtung (80) die erste Kupplung (K1) ihre ausgekuppelte Stellung und die zweite Kupplung (K2) ihre eingekuppelte Stellung einnimmt, so dass eine Drehung der Dosiereinrichtung (60) auf die Antriebseinrichtung (210, 230, 270, 310), aber nicht auf die Fördereinrichtung (90, 110, 120, 130, 140, 180) übertragbar ist,
und wobei eine Bewegung der Auslöseeinrichtung (80) von der Ruhestellung in die Auslösestellung zuerst ein Einkuppeln der ersten Kupplung (K1) und danach ein Auskuppeln der zweiten Kupplung (K2) bewirkt, so dass die Antriebseinrichtung (210, 230; 270, 310) freigegeben ist und die resultierende Antriebsbewegung auf die Fördereinrichtung (90, 110, 120, 130, 140, 180) übertragbar ist.

2. Vorrichtung nach Anspruch 1, wobei die Dosiereinrichtung (60) relativ zum Gehäuse (20) lösbar drehmomentenfest fixierbar ist.

3. Vorrichtung nach Anspruch 2, wobei die Dosiereinrichtung (60) über eine Ratschenverbindung relativ zum Gehäuse (20) lösbar drehmomentenfest fixierbar ist.

4. Vorrichtung nach Anspruch 3, wobei die Ratschenverbindung als Doppelrutschkupplung ausgebildet ist, die ein manuelles Verdrehen der Dosiereinrichtung (60) relativ zum Gehäuse (20) sowohl in einem ersten Drehsinn zum Erhöhen der zu verabreichenden Dosis als auch in einem entgegengesetzten Drehsinn zum Verringern der Dosis ermöglicht.

5. Vorrichtung nach Anspruch 4, wobei die Ratschenverbindung eine federbelastete Verzahnung umfasst, und wobei die Verzahnung asymmetrisch ausgestaltet ist.

6. Vorrichtung nach einem der Ansprüche 3 bis 5, wobei die Ratschenverbindung zwei sich axial gegenüberstehende Ratschenelemente (240, 260) umfasst, an deren Stirnseiten Zähne ausgebildet sind.

7. Vorrichtung nach Anspruch 6, wobei ein erstes der Rachtenelemente (260) als axial beweglicher, Richtung des zweiten Ratschenelements (240) federbelasteter Ratschenring ausgestaltet ist, der drehfest zum Dosierelement (690) angeordnet ist, und wobei das zweite Ratschenelement (240) dreh- und verschiebefest mit dem Gehäuse (20) verbunden ist.

8. Vorrichtung nach Anspruch 7, wobei der Ratschenring durch eine Mehrzahl von entlang dem Umfang des Ratschenrings angeordneten Federn federbelastet ist.

9. Vorrichtung nach einem der vorhergehenden Ansprüche, wobei die erste Kupplung (K1) durch Längsnuten auf einer Innenfläche eines Elements (230) der Antriebseinrichtung und Längsrippen auf einer Aussenfläche eines zum Eingangsglied der Fördereinrichtung drehfesten Elements (120) gebildet wird.

10. Vorrichtung nach einem der vorhergehenden Ansprüche, wobei die zweite Kupplung durch Längsnuten an einer Innenfläche eines Elements (250) der Dosiereinrichtung und Längsnuten an einer Aussenfläche eines Elements (270) der Antriebseinrichtung gebildet wird.

11. Vorrichtung nach einem der vorhergehenden Ansprüche,
- wobei zwischen dem Gehäuse (20) und der Fördereinrichtung (90, 110, 120, 130, 140, 180) eine dritte Kupplung (K3) ausgebildet ist, wobei das Gehäuse (20) und die Fördereinrichtung (90, 110, 120, 130, 140, 180) in einer eingekuppelten Stellung drehfest miteinander verbunden sind und in einer ausgekuppelten Stellung voneinander gelöst sind,
- wobei die dritte Kupplung (K3) in der Ruhestellung der Auslöseeinrichtung (80) ihre eingekuppelte Stellung einnimmt,
- und wobei eine Bewegung der Auslöseeinrichtung (80) von der Ruhestellung in die Auslösestellung zuerst ein Einkuppeln der ersten Kupplung (K1), danach ein Auskuppeln der zweiten Kupplung (K2) und danach ein Auskuppeln der dritten Kupplung (K3) bewirkt.

12. Vorrichtung nach Anspruch 11, wobei die dritte Kupplung (K3) durch Längsrippen auf der Aussenseite eines zum Eingangsglied der Fördereinrichtung drehfesten Elements (280) und Längsnuten auf der Innenseite eines zum Gehäuse drehfesten Elements (150) gebildet wird.

13. Vorrichtung nach einem der vorhergehenden Ansprüche, wobei die Auslöseeinrichtung (80) zwischen der Ruhestellung und der Auslösestellung in eine distale Richtung entlang der Vorschubachse bewegbar ist, und wobei jede der Kupplung (K1, K2, K3) eine Kupplungseingangsglied und ein Kupplungsausgangglied umfasst, die durch eine Bewegung der Auslöseeinrichtung eine relative Verschiebung gegeneinander erfahren und dadurch in Eingriff bzw. ausser Eingriff bringbar sind.

14. Vorrichtung nach Anspruch 13, wobei jede der Kupplung (K1, K2, K3) durch Längsnuten und Längsrippen auf radialen Innen- bzw. Aussenflächen des jeweiligen Kupplungsausgangsglieds gebildet wird.

15. Vorrichtung nach einem der vorhergehenden Ansprüche, wobei die Antriebseinrichtung entlang der Vorschubachse verschiebbar angeordnet ist, so dass eine Bewegung der Auslöseeinrichtung (80) von der Ruhestellung in die Auslösestellung eine Verschiebung der Antriebseinrichtung in eine distale Richtung bewirkt.

16. Vorrichtung nach Anspruch 14, wobei die Antriebseinrichtung entlang der Vorschubachse in einer der distalen Richtung entgegengesetzten proximalen Richtung federbelastet ist.

## Claims

1. A device for administering a fluid product,
- with a housing (20),
- with a conveying means (90, 110, 120, 130, 140, 180) for conveying the product from a reservoir (R, 40), wherein the conveying means comprises a rotatable input member (120) and a conveying element (90) movable along a feed axis, which can be driven by rotation of the input member (120),
- with a drive means (210, 230, 270, 310) to generate a rotary drive movement about the feed axis relative to the housing (20), wherein the drive means (210, 230, 270, 310) can be tensioned by a rotary tensioning movement,
- with a rotatable dosing means (60) for setting a dose of the product to be administered and tensioning of the drive means (210, 230, 270, 310) and
- with a triggering means (80), which can be moved from a rest position to a triggering position in the distal direction relative to the housing (20) and the dosing means (60) for triggering an administration,
- wherein a first clutch (K1) is formed between the drive means (210, 230, 270, 310) and the input member (120) of the conveying means (90, 110, 120, 130, 140, 180), wherein the drive means and the input member of the conveying means are interconnected in an engaged position in a rotatably fixed manner and are released from one another in a disengaged position,
- wherein a second clutch (K2) is formed between the dosing means (60) and the drive means (219, 230, 270, 310), wherein the dosing means and the drive means are interconnected in a rotatably fixed manner in an engaged position and are released from one another in a disengaged position,
- wherein the first clutch (K1) assumes its disengaged position in the rest position of the triggering means (80), and the second clutch (K2) assumes its engaged position, so that a rotation of the dosing means (60) can be transferred to the drive means (210, 230, 270, 310) but not to the conveying means (90, 110, 120, 130, 140, 180),
and wherein a movement of the triggering means (80) from the rest position to the triggering position first effects an engagement of the first clutch (K1) and thereafter a disengagement of the second clutch (K2), such that the drive means (210, 230, 270, 310) is released and the resulting drive movement can be transferred to the conveying means (90, 110, 120, 130, 140, 180).

2. The device according to claim 1, wherein the dosing means (60) can be detachably fastened in a torsionally fixed manner relative to the housing (20).

3. The device according to claim 2, wherein the dosing means (60) can be detachably fastened in a torsionally fixed manner relative to the housing (20) via a ratchet connection.

4. The device according to claim 3, wherein the ratchet connection is formed as a double slip clutch, which allows manual rotation of the dosing means (60) relative to the housing (20) both in a first direction of rotation to increase the dose to be administered and in an opposite direction of rotation to reduce the dose.

5. The device according to claim 4, wherein the ratchet connection comprises a spring-loaded gearing and wherein the gearing is configured asymmetrically.

6. The device according to one of the claims 3 to 5, wherein the ratchet connection comprises two axially opposing ratchet elements (240, 260) on the front sides of which teeth are formed.

7. The device according to claim 6, wherein a first of the ratchet elements (260) is configured as axially movable ratchet ring, spring-loaded in the direction of the second ratchet element (240), which is arranged in a rotatably fixed manner to the dosing element (690), and wherein the second ratchet element (240) is connected rotatably fixed and displacably fixed with the housing (20).

8. The device according to claim 7, wherein the ratchet ring is spring-loaded by way of a plurality of springs arranged along the periphery of the ratchet ring.

9. The device according to any one of the preceding claims, wherein the first clutch (K1) is formed by longitudinal grooves on an inner surface of an element (230) of the drive means, and longitudinal ridges on an outer surface of an element (120) rotatably fixed with respect to the input member of the conveying means.

10. The device according to any one of the preceding claims, wherein the second clutch is formed by longitudinal grooves on an inner surface of an element (250) of the dosing means and longitudinal grooves on an outer surface of an element (270) of the drive means.

11. The device according to any one of the preceding claims,
- wherein a third clutch (K3) is formed between the housing (20) and the conveying means (90, 110, 120, 130, 140, 180), wherein the housing (20) and the conveying means (90, 110, 120, 130, 140, 180) are interconnected in an engaged position in a rotatably fixed manner and are released from one another in a disengaged position,
- wherein the third clutch (K3) assumes its engaged position in the rest position of the triggering means (80),
- and wherein a movement of the triggering means (80) from the rest position to the triggering position first effects engagement of the first clutch (K1), thereafter disengagement of the second clutch (K2) and thereafter disengagement of the third clutch (K3).

12. The device according to claim 11, wherein the third clutch (K3) is formed by longitudinal ridges on the outer surface of an element (280) rotatably fixed with respect to the input member of the conveying means, and longitudinal grooves on the inner surface of an element (150) rotatably fixed with respect to the housing.

13. The device according to any one of the preceding claims, wherein the triggering means (80) is movable between the rest position and the triggering position in a distal direction along the feed axis, and wherein each of the clutches (K1, K2, K3) comprises a clutch input member and clutch output member, which experience a relative displacement relative to one another by a movement of the triggering means and thereby can be engaged or disengaged, -.

14. The device according to claim 13, wherein each of the clutches (K1, K2, K3) is formed by longitudinal grooves and longitudinal ridges on radial inner and outer surfaces respectively, of the respective output members of the clutch.

15. The device according to any one of the preceding claims, wherein the drive means is movably arranged along the feed axis, such that a movement of the triggering means (80) from rest position to the triggering position effects a displacement of the drive means in a distal direction.

16. The device according to claim 14, wherein the drive means is spring-loaded along the feed axis in a proximal direction opposite the distal direction.

## Revendications

1. Dispositif servant à administrer un produit fluide,
- avec un boîtier (20),
- avec un système de refoulement (90, 110, 120, 130, 140, 180) servant à refouler le produit hors d'un réservoir (R, 40), dans lequel le système de refoulement comprend un organe d'entrée (120) pouvant tourner et un élément de refoulement (90) pouvant être déplacé le long d'un axe d'avancement, lequel peut être entraîné par une rotation de l'organe d'entrée (120),
- avec un système d'entraînement (210, 230, 270, 310) servant à produire un mouvement de rotation d'entraînement autour de l'axe d'avancement par rapport au boîtier (20), dans lequel le système d'entraînement (210, 230, 270, 310) peut être serré par un mouvement de rotation de serrage,
- avec un système de dosage (60) pouvant tourner servant à régler une dose à administrer du produit et à serrer le système d'entraînement (210, 230, 270, 310) ; et
- avec un système de déclenchement (80), qui peut être déplacé depuis une position de repos dans une position de déclenchement dans une direction distale par rapport au boîtier (20) et au système de dosage (60) pour déclencher une administration,
- dans lequel un premier couplage (K1) est réalisé entre le système d'entraînement (210, 230, 270, 310) et l'organe d'entrée (120) du système de refoulement (90, 110, 120, 130, 140, 180), dans lequel le système d'entraînement et l'organe d'entrée du système de refoulement sont reliés l'un à l'autre de manière solidaire en rotation dans une position accouplée et détachés l'un de l'autre dans une position désaccouplée,
- dans lequel un deuxième couplage (K2) est réalisé entre le système de dosage (60) et le système d'entraînement (219, 230, 270, 310), dans lequel le système de dosage et le système d'entraînement sont reliés l'un à l'autre de manière solidaire en rotation dans une position accouplée et détachés l'un de l'autre dans une position désaccouplée,
- dans lequel, dans la position de repos du système de déclenchement (80), le premier couplage (K1) adopte sa position désaccouplée et le deuxième couplage (K2), sa position accouplée de sorte qu'une rotation du système de dosage (60) puisse être transférée sur le système d'entraînement (210, 230, 270, 310), toutefois pas sur le système de refoulement (90, 110, 120, 130, 140, 180),
et dans lequel un mouvement du système de déclenchement (80) depuis la position de repos dans la position de déclenchement entraîne dans un premier temps un accouplement du premier couplage (K1) et puis un désaccouplement du deuxième couplage (K2) de sorte que le système d'entraînement (210, 230 ; 270, 310) soit libéré et le mouvement d'entraînement en résultant puisse être transmis sur le système de refoulement (90, 110, 120, 130, 140, 180).

2. Dispositif selon la revendication 1, dans lequel le système de dosage (60) peut être fixé de manière solidaire en couple de rotation de manière amovible par rapport au boîtier (20).

3. Dispositif selon la revendication 2, dans lequel le système de dosage (60) peut être fixé de manière solidaire en couple de rotation de manière amovible par rapport au boîtier (20) par l'intermédiaire d'une liaison d'encliquetage.

4. Dispositif selon la revendication 3, dans lequel la liaison d'encliquetage est réalisée en tant que double couplage glissant, qui permet une rotation manuelle du système de dosage (60) par rapport au boîtier (20) à la fois dans un premier sens de rotation pour augmenter la dose à administrer et dans un sens de rotation opposé pour réduire la dose.

5. Dispositif selon la revendication 4, dans lequel la liaison d'encliquetage comprend une denture soumise à ressort, et dans lequel la denture est configurée de manière asymétrique.

6. Dispositif selon l'une quelconque des revendications 3 à 5, dans lequel la liaison d'encliquetage comprend deux éléments d'encliquetage (240, 260) se faisant face de manière axiale, au niveau des côtés frontaux desquels sont réalisées des dents.

7. Dispositif selon la revendication 6, dans lequel un premier des éléments d'encliquetage (260) est configuré en tant que bague d'encliquetage axialement mobile, contrainte par ressort en direction du deuxième élément d'encliquetage (240), qui est disposée de manière solidaire en rotation par rapport à l'élément de dosage (690), et dans lequel le deuxième élément d'encliquetage (240) est relié au boîtier (20) de manière solidaire à la rotation et au coulissement.

8. Dispositif selon la revendication 7, dans lequel la bague d'encliquetage est soumise à la contrainte d'un ressort par une multitude de ressorts disposés le long du contour de la bague d'encliquetage.

9. Dispositif selon l'une quelconque des revendications précédentes, dans lequel le premier couplage (K1) est formé par des rainures longitudinales sur une surface intérieure d'un élément (230) du système d'entraînement et des nervures longitudinales sur une surface extérieure d'un élément (120) solidaire en rotation par rapport à l'organe d'entrée du système de refoulement.

10. Dispositif selon l'une quelconque des revendications précédentes, dans lequel le deuxième couplage est formé par des rainures longitudinales au niveau d'une surface intérieure d'un élément (250) du système de dosage et des rainures longitudinales au niveau d'une surface extérieure d'un élément (270) du système d'entraînement.

11. Dispositif selon l'une quelconque des revendications précédentes,
- dans lequel est réalisé, entre le boîtier (20) et le système de refoulement (90, 110, 120, 130, 140, 180), un troisième couplage (K3), dans lequel le boîtier (20) et le système de refoulement (90, 110, 120, 130, 140, 180) sont reliés l'un à l'autre de manière solidaire en rotation dans une position accouplée et détachés l'un de l'autre dans une position désaccouplée,
- dans lequel le troisième couplage (K3) adopte, dans la position de repos du système de déclenchement (80), sa position accouplée,
- et dans lequel un mouvement du système de déclenchement (80) depuis la position de repos dans la position de déclenchement entraîne dans un premier temps un accouplement du premier couplage (K1), puis un désaccouplement du deuxième couplage (K2) et puis un désaccouplement du troisième couplage (K3).

12. Dispositif selon la revendication 11, dans lequel le troisième couplage (K3) est formé par des nervures longitudinales sur le côté extérieur d'un élément (280) solidaire en rotation par rapport à l'organe d'entrée du système de refoulement et des rainures longitudinales sur le côté intérieur d'un élément (150) solidaire en rotation par rapport au boîtier.

13. Dispositif selon l'une quelconque des revendications précédentes, dans lequel le système de déclenchement (80) entre la position de repos et la position de déclenchement peut être déplacé dans une direction distale le long de l'axe d'avancement, et dans lequel chacun des couplages (K1, K2, K3) comprend un organe d'entrée de couplage et un organe de sortie de couplage, qui peuvent subir un coulissement relatif les uns par rapport aux autres par un mouvement du système de déclenchement et peuvent de ce fait être mis en prise ou hors prise.

14. Dispositif selon la revendication 13, dans lequel chacun des couplages (K1, K2, K3) est formé par des rainures longitudinales et des nervures longitudinales sur des surfaces intérieures ou extérieures radiales de l'organe de sortie de couplage respectif.

15. Dispositif selon l'une quelconque des revendications précédentes, dans lequel le système d'entraînement est disposé de manière à pouvoir coulisser le long de l'axe d'avancement de sorte qu'un mouvement du système de déclenchement (80) depuis la position de repos dans la position de déclenchement entraîne un coulissement du système d'entraînement dans une direction distale.

16. Dispositif selon la revendication 14, dans lequel le système d'entraînement est contraint par des ressorts le long de l'axe d'avancement dans une direction proximale opposée à la direction distale.
